(19) Europäisches Patentamt
European Patent Office
Office européen des brevets

(11) EP 1 543 117 B1

(12) **EUROPEAN PATENT SPECIFICATION**

(45) Date of publication and mention of the grant of the patent:
**30.09.2009 Bulletin 2009/40**

(21) Application number: **03744218.3**

(22) Date of filing: **05.03.2003**

(51) Int Cl.:
*C12N 9/20* (2006.01)

(86) International application number:
**PCT/US2003/006908**

(87) International publication number:
**WO 2003/076580 (18.09.2003 Gazette 2003/38)**

(54) **HIGH THROUGHPUT MUTAGENESIS SCREENING METHOD**

MUTAGENESE-SCREENINGVERFAHREN MIT HOHEM DURCHSATZ

METHODE DE CRIBLAGE DE MUTAGENESE HAUTE DENSIT

(84) Designated Contracting States:
**AT BE BG CH CY CZ DE DK EE ES FI FR GB GR HU IE IT LI LU MC NL PT RO SE SI SK TR**

(30) Priority: **05.03.2002 US 91912**
**05.03.2002 US 92227**
**05.03.2002 US 362372 P**
**15.08.2002 US 403921 P**

(43) Date of publication of application:
**22.06.2005 Bulletin 2005/25**

(73) Proprietor: **GENENCOR INTERNATIONAL, INC.**
**Palo Alto, California 94304 (US)**

(72) Inventors:
- **BOTT, Richard, R.**
  **Burlingame, CA 94010 (US)**
- **KELLIS, James, T., Jr.**
  **Portola Valley, CA 94028 (US)**
- **MORRISON, Thomas, B.**
  **Winchester, MA 01890 (US)**

(74) Representative: **Kiddle, Simon John**
**Mewburn Ellis LLP**
**33 Gutter Lane**
**London**
**EC2V 8AS (GB)**

(56) References cited:
**EP-A2- 0 268 452** **US-A- 5 352 594**

- **SAGT C M J ET AL: "Impaired secretion of a hydrophobic cutinase by Saccharomyces cerevisiae correlates with an increased association with immunoglobulin heavy-chain binding protein (BiP)" APPLIED AND ENVIRONMENTAL MICROBIOLOGY, WASHINGTON,DC, US, vol. 64, no. 1, January 1998 (1998-01), pages 316-324, XP002088542 ISSN: 0099-2240**
- **LONGHI S ET AL: "DYNAMICS OF FUSARIUM SOLANI CUTINASE INVESTIGATED THROUGH STRUCTURAL COMPARISON AMONG DIFFERENT CRYSTAL FORMS OF ITS VARIANTS" PROTEINS: STRUCTURE, FUNCTION AND GENETICS, ALAN R. LISS, US, vol. 26, 1996, pages 442-458, XP002926640 ISSN: 0887-3585**
- **CARVALHO C M L ET AL: "Cutinase structure, function and biocatalytic applications" EJB ELECTRONIC JOURNAL OF BIOTECHNOLOGY, CONICYT, SANTIAGO, CL, vol. 1, no. 3, 1998, pages 160-173, XP002320332 ISSN: 0717-3458**
- **GRAUSCHOPF ULLA ET AL: "Why is DsbA such an oxidizing disulfide catalyst?" CELL, vol. 83, no. 6, 1995, pages 947-955, XP002418766 ISSN: 0092-8674**
- **MOORE J C ET AL: "STRATEGIES FOR THE IN VITRO EVOLUTION OF PROTEIN FUNCTION: ENZYME EVOLUTION BY RANDOM RECOMBINATION OF IMPROVED SEQUENCES" JOURNAL OF MOLECULAR BIOLOGY, LONDON, GB, vol. 272, no. 3, 26 September 1997 (1997-09-26), pages 336-347, XP000892797 ISSN: 0022-2836**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 543 117 B1

- **BOSTON M. ET AL.: 'Structure and function of engineered Pseudomonas mendocina lipase' METHODS ENZYMOL. vol. 284, 1997, pages 298 - 317, XP008044067**

## Description

### FIELD OF THE INVENTION

The invention provides cutinase variants that provide improved stability and activity compared with the wild type cutinase.

### BACKGROUND OF THE INVENTION

Various mutagenesis procedures are used in the modification of proteins to obtain proteins with desired properties and/or capabilities. In most of these methods, the nucleotide sequence of a cloned gene encoding a protein is mutated and the modified gene is expressed to produce mutants, which are then typically screened for activities of interest. Then, the activities of the mutant proteins are usually compared with those of wild-type proteins.
A number of mutagenesis methods are known in the art. One general method involves site-saturation mutagenesis, which results in a mutated gene population that consists of otherwise identical genes, but includes randomly introduced codons. The random introduced codon can code for any amino acid, and the position is said to be "saturated" (Airaksinen and Hovi, Nucleic Acids.Res.. 26:576-581[1998]). There are several different site-saturation methods known to those in the art.
Because most mutagenesis methods provide a great number of amino acid mutation options, screening of a large number of variants generally is required to produce a desired protein property. Generally, screening is repeated multiple times in order to produce a beneficial variant of interest. Thus, there is a continuing need in the art for mutagenesis and screening methods that provide relative ease of use as well as being efficient in terms of protocols and data analysis, in order to obtain desired protein engineering results.
EP0268452 describes a cutinase from P. putida ATCC53552, now classified as P. mendocina.

### SUMMARY OF THE INVENTION

The present application describes mutagenesis methods for protein engineering, In particular, the present application describes mutagenesis methods that include feedback adjustment from systematic result evaluation. More specifically, the application describes site-saturation mutagenesis methods that screen for variants with one or more desirable protein properties and evaluates screening results to provide feedback for repeat screening and construction of new libraries. The invention provides cutinase variants that provide improved stability and activity compared with the wild type cutinase.

The high throughput mutagenesis screening methods select sites for saturation scanning using protein structural considerations. In preferred embodiments, site-saturation libraries are created and screened using assays selected to identify clones having protein properties of interest. The mutation sites are categorized based on their properties of interest, and trends, if any, are identified. A variant or multiple variants having at least one desired property is/are selected for additional library creation procedures. These additional libraries are created using feedback from the determined categories. One set of additional libraries repeats construction of the previous libraries using the feedback. A second set of additional libraries represents new libraries created at sites that are expected to be beneficial based on the feedback. In some embodiments these categories are coded (*i.e.*, using color or other indicia), to allow easy identification of trends.

The present invention provides numerous cutinase variants. These variants comprise a substitution of one or more amino acids at residue positions corresponding to sites 34, 73, 75, 77, 78, 79, 80, 99, 164, 191-196, 219, 223 and 225 of *Pseudomonas mendocina* cutinase SEQ ID NO: 2, wherein said cutinase variant is thermostable and has hydrolytic activity an polyester. In some particularly preferred embodiments, the variants provide improved activity under various conditions and/or improved stability compared with the wild type cutinase. In some embodiments, the present invention provides cutinase variants of *Pseudomonas mendocina* cutinase SEQ ID NO: 2, wherein the variants comprise substitution of Phe 194 with Pro, substitution of Ser 219 with Gly, and one or more additional substitutions in at least one amino acid position selected from the amino acid positions 54, 78, 81, 191, 196, 200, 204, and 263.
In some embodiments, the variants have amino acid position 54 substituted with Ser, Glu, or Thr. In other embodiments, the amino acid position 78 is substituted with Val. In further embodiments, the amino acid position 81 is substituted with Ser. In still further embodiments, amino acid position 191 | is substituted with Asn or His. In other embodiments, amino acid position 196 is substituted with Leu or Ala. In additional embodiments, amino acid position 200 is substituted with Leu. In yet additional embodiments, amino acid position 204 is substituted with Leu. In further embodiments amino acid position 263 is substituted with Pro.
The present invention also provides cutinase variants comprising a substitution of one or more amino acids at residue positions corresponding to sites 34, 73, 75, 77, 78, 79, 80, 99, 164, 191-196, 219, 223 and 225 of *Pseudomonas*

*mendocina* cutinase SEQ ID NO: 2, wherein the cutinase variants have polyesterase activity. In some embodiments, the cutinase variants comprise the substitution of GLY 73 with an amino acid selected from the group consisting of Phe, Lys, Leu, and Val, and wherein the cutinase variant has increased stability as compared to wild-type cutinase. In other embodiments, the cutinase variant comprises the substitution of Thr 191 with an amino acid selected from the group consisting of His, Leu, and Tyr, and wherein the cutinase variant has increased stability as compared to wild-type cutinase. In alternative embodiments, the cutinase variant comprises the substitution of Thr 78 with an amino acid selected from the group consisting of Ala, Leu and Val, and wherein the cutinase variant has increased stability as compared to wild-type cutinase. In some embodiments, the cutinase variant comprises the substitution of Tyr 164 with Phe, and wherein the cutinase variant has increased stability as compared to wild-type cutinase. In still other embodiments, the cutinase variant comprises the substitution of Tyr 196 with an amino acid selected from the group consisting of Ala, Leu, and Pro, and wherein the cutinase variant has increased stability as compared to wild-type cutinase. In yet additional embodiments, the cutinase variant comprises the substitution of Phe 194 and substitution of Ser 219, and wherein the cutinase variant has increased stability as compared to wild-type cutinase. In further embodiments, the Phe at position 194 is substituted with an amino acid selected from the group consisting of Ala, His, Lys, Leu, Asn, Pro, and Gly, and wherein Ser219 is substituted with Gly. In some particularly preferred embodiments, the cutinase variant has increased polyesterase activity as compared to wild-type cutinase. In some embodiments, the cutinase variant comprises the substitution of Ala 80 with Glu, and wherein the cutinase variant has increased stability as compared to wild-type cutinase. In additional preferred embodiments, the cutinase variant comprises the substitution of Phe 194 with Ile, and the cutinase variant has increased polyesterase activity as compared to wild-type cutinase. In still further embodiments, the cutinase variant comprises the substitution of Phe 194 with an amino acid selected from the group consisting of Lys and Leu, and the substitution of Ser 219 with Gly, and the cutinase variant has increased polyesterase activity as compared to wild-type cutinase. In yet other embodiments, the cutinase variant comprises the substitution of Phe 194 with Asn, and wherein the cutinase variant has increased polyesterase activity as compared to wild-type cutinase. In further embodiments, the cutinase variant comprises the substitution of Phe 194 with an amino acid selected from the group consisting of Asn, Pro, and Ser, and the substitution of Ser 205 with Gly, and wherein the cutinase variant has increased polyesterase activity as compared to wild-type cutinase. In yet additional embodiments, the cutinase variant comprises the substitution of Gly 73 with an amino acid selected from the group consisting of Phe and Leu, and wherein the cutinase variant has increased polyesterase activity as compared to wild-type cutinase. In further embodiments, the cutinase variant comprises the substitution of Gly 75 with an amino acid selected from the group consisting of Asp and Glu, and wherein the cutinase variant has increased polyesterase activity as compared to wild-type cutinase. In some embodiments, the variant comprises the following substitutions Ile 192 with Met; Phe 194 with Val; and Ser 219 with Gly. In particularly preferred embodiments, these cutinase variants have increased polyesterase activity as compared to wild-type cutinase.

In still further embodiment, the cutinase variant comprises the substitution of Ser 219 with Asn, and substitution of Phe 221; with Leu, and has increased polyesterase activity as compared to wild-type cutinase.

In additional embodiments, the cutinase variant comprises the substitution of Ser 99|with Lys, and has increased polyesterase activity as compared to wild-type cutinase. In further embodiments, the cutinase variant comprises the substitution of Thr 191 with an amino acid selected from the group consisting of His, Leu, and Tyr, and has increased polyesterase activity as compared to wild-type cutinase. In alternative embodiments, the cutinase variant comprises the substitution of Thr 78 with Leu, and has increased polyesterase activity as compared to wild-type cutinase. In further embodiments, the cutinase variant comprises the substitution of Tyr 196 with an amino acid selected from the group consisting of Ala and Pro, and has increased polyesterase activity as compared to wild-type cutinase. In some embodiments, the cutinase variant comprises the substitution of Phe 1941 with an amino acid selected from the group consisting of Ile, Leu, Asn, and Pro, and has increased stability as compared to wild-type cutinase. In other embodiments, the cutinase variant comprises the substitution of Arg 204 with Leu, and has increased stability as compared to wild-type cutinase. In additional embodiments, the cutinase variant comprises the substitution of Arg 204 with Leu, and has increased polyesterase activity as compared to wild-type cutinase. In yet further embodiments, the cutinase variant comprises the substitution of Leu,263 with Pro, and has increased stability as compared to wild-type cutinase. In other embodiments, the cutinase variant comprises the substitution of Leu 263 with Pro, and has increased polyesterase activity as compared to wild-type cutinase. In some embodiments, the mutations described above are present in combination, while in other embodiments, they are present as single mutations.

The present invention further provides cutinase variants comprising a substitution of one or more amino acids at residue positions corresponding to sites 34, 73, 75, 77, 78, 79, 80, 99, 164, 191-196, 219,223 and 225 of *Pseudomonas mendocina* cutinase SEQ ID NO:: 2, wherein the cutinase variant is thermostable and has hydrolytic activity on polyester.

The present application also describes methods for obtaining a variant having one or more desired protein properties comprising the steps of: selecting amino acid sites in a protein for mutation; performing mutagenesis at the selected mutation sites to create a library; screening the library for variants having one or more desired protein properties; grading the mutation sites of the variants for the one or more desired protein properties; selecting one or more variants having a desirable grade as a template for, with feedback from the grading, creating and screening additional libraries, whereby the method utilizes cooperative mutations to obtained a variant having at least two mutations. In some embodiments, the mutagenesis step is performed by site-saturation mutagenesis and wherein selecting amino acid sites is performed by utilizing protein structural considerations. In additional embodiments, the steps of creating and screening additional libraries are performed by repeating site-saturation mutagenesis at mutation sites having desirable grades and performing site-saturation mutagenesis at new sites on new libraries. In further embodiments, the step of performing repeating site-saturation is performed by selecting sites located near mutation sites having desirable grades. In yet additional embodiments, protein structural considerations comprise such factors as the binding site location, three-dimensional structure, amino acid sequence, nature of chemical reaction, or nature of chemical binding. In some preferred embodiments, the protein property comprises an enzyme property. In some particularly preferred embodiments, the enzyme property comprises at least one property selected from the group consisting of catalysis, binding, and stability. In alternative embodiments, the step of screening for one or more variants is performed by selecting and conducting assays for the one or more protein properties of interest. In yet other embodiments, grading is performed by identifying trends. In some preferred embodiments, the step of identifying trends is performed by plotting a spatial distribution of graded sites on a three-dimensional rendition of the protein. In some further preferred embodiments, the step of identifying trends is performed by plotting amino acid mutation identities. In yet other embodiments, the step of identifying trends is performed by plotting a distribution of graded mutation sites. In additional embodiments, the steps of creating and screening additional libraries are performed by screening the additional libraries for the desired protein properties and repeating site-saturation mutagenesis until a desired protein property goal is attained.

The present application also describes methods or obtaining a variant enzyme having one or more desired properties comprising: utilizing a three-dimensional rendition of the enzyme to select mutation sites based on the amino acids; performing site-saturation mutagenesis at the selected mutation sites to create a library; screening the library for variants having one or more desired properties; grading the mutation sites of the variants for one or more desired properties; selecting one or more variants having a desirable grade as a template; using the template and feedback to repeat site-saturation mutagenesis at mutation sites having desirable grades and to perform site-saturation mutagenesis on new libraries at new sites. In yet additional embodiments, one or more desired properties are selected from the group consisting of substrate activity, thermostability, stability relative to reaction environment, ionic strength range of stability, pressure stability, and pH range of stability. In some preferred embodiments, one or more desired properties is selected from the group consisting of substrate activity and thermostability. In some particularly preferred embodiments, the enzyme is cutinase.

The present application also describes processes (*i.e.*, methods) for the production of a cutinase variant with hydrolytic activity on polyester, wherein the cutinase is a variant of *Pseudomonas* cutinase, the process comprising: utilizing a three-dimensional model to select for mutation amino acid sites likely to demonstrate hydrolytic activity; performing site-saturation mutagenesis at the selected mutation amino acid sites to create a library having variants with mutated sites; screening the library for variants having mutated sites, using assays to detect polyesterase activity and thermostability; grading the mutated sites as beneficial, neutral or detrimental for both polyesterase activity and thermostability; selecting a variant having at least one beneficial grade; creating new and repeat libraries using the selected variant and feedback from the grading.

## BRIEF DESCRIPTION OF THE DRAWINGS

Figure 1 provides a diagrammatic view illustrating the general principle of the invention.

Figure 2 provides a graph showing the relationship between polyesterase activity and protein concentration, as determined by TCA assay for several different *Bacillus subtilis* transformants.

Figure 3 provides a graph showing the polyesterase activity of "Library A" cutinase variants in two site-saturation libraries relative to TCA assay results. Wild-type enzyme controls are shown as well.

Figure 4 provides a graph showing the polyesterase activity of "Library B" cutinase variants in two site-saturation libraries relative to TCA assay results. Wild-type enzyme controls are shown as well.

Figure 5 provides a graph showing the thermal stability in laundry detergent of "Library A" cutinase variants in two site-saturation libraries relative to pNB activity results. Wild-type enzyme controls are also shown.

Figure 6 provides a graph showing the thermal stability in laundry detergent of "Library B" cutinase variants in two site-saturation libraries relative to pNB activity results. Wild-type enzyme controls are also shown.

Figure 7 provides a diagram showing the 3-dimensional structure of a protein showing the catalytic triad (dark color) active site face of *Pseudomonas mendocina* cutinase.

Figure 8 provides an alternate rendition of Figure 7. In this Figure, the initial target amino acids used for the site-saturation mutagenesis procedure of the present invention are shown in a darker color.

Figure 9 provides an alternate rendition of Figure 7. In this Figure, sites demonstrating at least 2% of variants with improved enzyme activity are shown in a darker color.

Figure 10 provides another alternate rendition of Figure 7. In this Figure, sites in which the majority of mutations had no effect on the enzyme activity are shown in dark color.

Figure 11 provides another rendition of Figure 7. In this Figure, sites in which the majority of mutations had a detrimental effect on the enzyme-activity are shown in dark color.

Figure 12 provides another rendition of Figure 7. In this Figure, sites demonstrating at least 2% of variants with improved protein stability are shown in dark color.

Figure 13 shows yet another rendition of Figure 7. In this Figure, sites in which the majority of mutations had no effect on protein stability are shown in dark color.

Figure 14 provides another rendition of Figure 7. In this Figure, sites in which the majority of mutations had a detrimental effect on protein stability are shown in dark color.

Figure 15 provides a graph showing the activity of a second generation variant in comparison with the wild type enzyme.

Figure 16 provides a graph showing the increased thermostability of a second generation-variant versus the wild type.

Figure 17 provides the nucleotide sequence (SEQ ID NO:1) of the parent *P. mendocina cutinase* expressed in *Bacillus subtilis* #3934.

Figure 18 provides the amino acid (SEQ ID NO:2) and DNA sequence (SEQ ID NO:1) for the parent *P. mendocina* cutinase.

## DESCRIPTION OF THE INVENTION

The present application describes mutagenesis methods for protein engineering. In particular, the present application describes mutagenesis methods that include feedback adjustment from systematic result evaluation. More specifically, the application describes site-saturation mutagenesis methods that screen for variants with one or more desirable protein properties and evaluates screening results to provide feedback for repeat screening and construction of new libraries. The invention provides cutinase variants that provide improved stability and activity compared with the wild type cutinase.

The present application describes scanning and site-saturation mutagenesis methods for producing improved protein variants using as few as two sequential amino acid substitutions. In preferred embodiments, the methods include feedback adjustment from systematic screening result evaluation for multiple protein properties following amino acid substitution. The method both identifies and grades amino acid substitutions and the mutation sites of the substitutions based upon the results of assays selected to detect at least one desired, improved protein property. The method further correlates the mutation sites and the specific mutations with the assay results, facilitating the identification of trends and enabling construction of new molecular libraries and graded identification of mutation sites for further improvement. The method also involves repeated mutagenesis of the initial libraries, resulting in cooperative mutations that improve protein properties with as few as two mutations. New libraries also are created at new sites selected with grading feedback.

The general methods are set out generally in Figure 1. In preferred embodiments, sites are selected for saturation based upon protein structural information and/or any hypotheses related to structural-functional relationships at step 2 (*See*, Figure 1), and site-saturation libraries are created at step 4 (*See*, Figure 1). The created libraries are then screened at step 6 (*See*, Figure 1) for their property(ies) of interest, and the screening results are used to categorize sites at step 8 (*See*, Figure 1). For example, in some preferred embodiments, the sites are ranked as "beneficial," "neutral," or "detrimental," while in other embodiments, other categories are designated as desired. At step 10 (*See*, Figure 1), an improved variant is selected based on the results obtained at step 8 (*See*, Figure 1), and the variant is used as a template for additional library construction. Library construction at step 12 (*See*, Figure 1) utilizes the template variant from step 10 (*See*, Figure 1) and creates new sites expected to be beneficial based on feedback from the categorized results from step 8 (*See*, Figure 1). In addition to new libraries, the improved variant from step 10 (*See*, Figure 1) is used to repeat the previous libraries at the beneficial sites at step 14 using feedback from the categorized results from step 8 (*See*, Figure 1).

The site-saturation scanning mutagenesis methods are conducted in an iterative fashion, with feedback adjustment, and in some embodiments, are broken down into the following four sections: site-saturation mutagenesis; activity screening; systematic result evaluation; new library construction/repeat site-saturation mutagenesis of previous libraries.

### Definitions

Unless defined otherwise herein, all technical and scientific terms used herein have the same meaning as commonly understood by one of ordinary skill in the art to which this invention pertains. For example, Singleton and Sainsbury, Dictionary of Microbiology and Molecular Biology, 2d Ed., John Wiley and Sons, NY (1994); and Hale and Marham, The Harper Collins Dictionary of Biology, Harper Perennial, NY (1991) provide those of skill in the art with a general dictionaries of many of the terms used in the invention. Although any methods and materials similar or equivalent to those described herein find use in the practice of the present invention, the preferred methods and materials are described herein. Accordingly, the terms defined immediately below are more fully described by reference to the Specification as a whole. Also, as used herein, the singular "a", "an" and "the" includes the plural reference unless the context clearly indicates otherwise.

As used herein, *"Pseudomonas"* refers to all of the members of the bacterial genus *Pseudomonas,* including but not limited to *P. fluorescens, P.synxantha, P. mendocina, P. aeruginosa, P. aureofaciens,* and any other species within the genus *Pseudomonas.* It is intended that the term encompass wild-type strains, as well as mutant and recombinant strains. It is also intended that the genus include species that have been reclassified to another genus.

As used herein, "the genus *Bacillus*" includes all members known to those of skill in the art, including but not limited to *B. subtilis, B. licheniformis, B. lentus, B. brevis, B. stearothermophilus, B. alkalophilus, B. amyloliquefaciens, B. clausii, B. halodurans, B. megaterium, B. coagulans, B. circulans, B. lautus,* and *B. thuringiensis.* It is recognized that the genus *Bacillus* continues to undergo taxonomical reorganization. Thus, it is intended that the genus include species that have been reclassified, including but not limited to such organisms as *B. stearothermophilus,* which is now named *"Geobacillus stearothermophilus."* The production of resistant endospores in the presence of oxygen is considered the defining feature of the genus *Bacillus,* although this characteristic also applies to the recently named *Alicyclobacillus, Amphibacillus, Aneurinibacillus, Anoxybacillus, Brevibacillus, Filobacillus, Gracilibacillus, Halobacillus, Paenibacillus, Salibacillus, Thermobacillus, Ureibacillus,* and *Virgibacillus.* It is intended that the term encompass wild-type strains, as well as mutant and recombinant strains.

As used herein, "wild-type" and "native" proteins are those found in nature. The terms "wild-type sequence," and "wild-type gene" are used interchangeably herein, to refer to a sequence that is native or naturally occurring in a host cell. In some embodiments, the wild-type sequence refers to a sequence of interest that is the starting point of a protein engineering project. The genes encoding the naturally-occurring (*i.e.*, precursor) protein may be obtained in accord with the general methods known to those skilled in the art. The methods generally comprise synthesizing labeled probes having putative sequences encoding regions of the protein of interest, preparing genomic libraries from organisms expressing the protein, and screening the libraries for the gene of interest by hybridization to the probes. Positively hybridizing clones are then mapped and sequenced.

As used herein, "recombinant protein," refers to a protein in which the DNA sequence encoding the protein has been modified to produce a variant (or mutant) DNA sequence which encodes the substitution, deletion or insertion of one or more amino acids in the naturally-occurring amino acid sequence. Suitable methods to produce such modification, and which may be combined with those disclosed herein, include, but are not limited to those disclosed in US Patent 4,760,025 (US RE 34,606), US Patent 5,204,015 and US Patent 5,185,258,

As used herein, "protein" refers to any composition comprised of amino acids and recognized as a protein by those of skill in the art. The terms "protein," "peptide" and polypeptide are used interchangeably herein. Amino acids may be referred to by their complete names (*e.g.*, alanine) or by the accepted one letter (*e.g.*, A), or three letter (*e.g.*, ala) abbreviations. Wherein a peptide is a portion of a protein, those skill in the art understand the use of the term in context. The term "protein" encompasses mature forms of proteins, as well as the pro- and prepro-forms of related proteins. Prepro forms of proteins comprise the mature form of the protein having a prosequence operably linked to the amino terminus of the protein, and a "pre-" or "signal" sequence operably linked to the amino terminus of the prosequence.

In some preferred embodiments, the term protein refers to natural, synthetic, and engineered enzymes such as oxidoreductases, transferases, isomerases, ligases, hydrolases; antibodies; polypeptides; peptides; hormones; cytokines; growth factors; other biological modulators; protein-based diagnostic reagents; and biosensors. Examples of enzymes that may be enhanced by the method of the invention include, but are not limited to, proteases, carbohydrases, lipases, peroxidases, cellulases, and dioxygenases.

As used herein, the term "derivative" refers to a protein (*e.g.*, a cutinase) which is derived from a precursor protein (*e.g.*, the native cutinase) by addition of one or more amino acids to either or both the C- and N-terminal end(s), substitution of one or more amino acids at one or a number of different sites in the amino acid sequence, and/or deletion of one or more amino acids at either or both ends of the protein or at one or more sites in the amino acid sequence, and/or insertion of one or more amino acids at one or more sites in the amino acid sequence. The preparation of a cutinase derivative is preferably achieved by modifying a DNA sequence which encodes for the native protein, transformation of that DNA sequence into a suitable host, and expression of the modified DNA sequence to form the derivative cutinase.

Related (and derivative) proteins include "variant proteins." In preferred embodiments, variant proteins differ from a parent protein and one another by a small number of amino acid residues. The number of differing amino acid residues may be one or more, preferably 1, 2, 3, 4, 5, 10, 15, 20, 30, 40, 50, or more amino acid residues. In one preferred embodiment, the number of different amino acids between variants is between 1 and 10. In particularly preferred embodiments, related proteins and particularly variant proteins comprise at least 50%, 60%, 65%. 70%, 75%, 80%, 85%, 90%, 95%, 97%, 98%, or 99% amino acid sequence identity.

As used herein, "protein properties" refers to protein structural integrity and/or function, including binding and/or catalysis relative to one or more conditions or substrates, protein activity including but not limited to binding and catalysis; protein stability relative to exposure to selected conditions, protein stability including but not limited to thermal stability, pH range stability, ionic strength range stability, pressure stability; and/or reaction substance environment stability, such as stability in organic solvents.

As used herein, "protein structural considerations" refers to available information about the physical structure of the protein of interest, including but not limited to substrate binding site location, three-dimensional structure, amino acid sequences; and, the chemical nature of the reaction or binding event to be addressed by mutagenesis of the protein.

The use of protein structural considerations is illustrated by the following two examples which are not meant to be limiting. When both the site of substrate binding and the three-dimensional structure are known, site-saturation mutagenesis may be limited to a substrate binding site if the target property is catalytic activity. When only the amino acid sequence of the target protein is known, mutagenesis sites can be selected by comparing the known sequence to homologous sequences to allow selection of suspected functionally important sites for mutagenesis. Both examples further include consideration of the nature of the problem to be solved (*i.e.* alteration of catalytic activity, binding, stability, etc.).

As used herein, "site-saturation mutagenesis" refers to a method of mutagenesis wherein oligonucleotide primers are randomized at the site of interest using the triplet sequence NNG/C, where N is a mixture of all 4 bases. For each site selected, the amino acid is randomly replaced with all 19 possible alternatives. Site-saturation mutagenesis is not limited to mutations produced by a single codon substitution. In some preferred embodiments, mutant DNA sequences are generated with site saturation mutagenesis in at least one codon. In other preferred embodiments, site saturation mutagenesis is performed for two or more codons. In a further embodiment, mutant DNA sequences have more than 40%, more than 45%, more than 50%, more than 55%, more than 60%, more than 65%, more than 70%, more than 75%, more than 80%, more than 85%, more than 90%, more than 95%, or more than 98% homology with the wild-type sequence. Alternatively, mutant DNA may be generated *in vivo* using any known mutagenic procedure (*e.g.*, radiation, nitrosoguanidine, etc.). The DNA construct sequences may be wild-type, mutant or modified. In addition, the sequences may be homologous or heterologous.

As used herein, the term "targeted randomization" refers to a process that produces a plurality of sequences where one or several positions have been randomized. In some embodiments, randomization is complete (*i.e.*, all four nucleotides, A, T, G, and C can occur at a randomized position. In alternative embodiments, randomization of a nucleotide is limited to a subset of the four nucleotides. Targeted randomization can be applied to one or several codons of a sequence, coding for one or several proteins of interest. When expressed, the resulting libraries produce protein populations in which one or more amino acid positions can contain a mixture of all 20 amino acids or a subset of amino acids, as determined by the randomization scheme of the randomized codon. In some embodiments, the individual members of a population resulting from targeted randomization differ in the number of amino acids, due to targeted or random insertion or deletion of codons. In further embodiments, synthetic amino acids are included in the protein populations produced.

As used herein, "corresponding to," refers to a residue at the enumerated position in a protein or peptide, or a residue that is analogous, homologous, or equivalent to an enumerated residue in another protein or peptide.

As used herein, "corresponding region" generally refers to an analogous position within related proteins or a parent protein.

As used herein, the term "analogous sequence" refers to a sequence within a protein that provides similar function, tertiary structure, and/or conserved residues as the protein of interest. In particularly preferred embodiments, the analogous sequence involves sequence(s) at or near an epitope. For example, in epitope regions that contain an alpha helix or a beta sheet structure, the replacement amino acids in the analogous sequence preferably maintain the same specific structure. The term also refers to nucleotide sequences, as well as amino acid sequences.

As used herein, "homologous protein" refers to a protein (*e.g.*, cutinase) that has similar catalytic action, structure, antigenic, and/or immunogenic response as the protein (*i.e.*, cutinase) of interest. It is not intended that a homolog and a protein (*e.g.*, cutinase) of interest be necessarily related evolutionarily. Thus, it is intended that the term encompass the same functional protein obtained from different species. In some preferred embodiments, it is desirable to identify a homolog that has a tertiary and/or primary structure similar to the protein of interest, as replacement for the epitope in the protein of interest with an analogous segment from the homolog will reduce the disruptiveness of the change. Thus, in most cases, closely homologous proteins provide the most desirable sources of epitope substitutions. Alternatively, it is advantageous to look to human analogs for a given protein.

As used herein, "homologous genes" refers to at least a pair of genes from different, but usually related species, which correspond to each other and which are identical or very similar to each other. The term encompasses genes that are separated by speciation.(*i.e.*, the development of new species) (*e.g.*, orthologous genes), as well as genes that have been separated by genetic duplication (*e.g.*, paralogous genes).

As used herein, "ortholog" and "orthologous genes" refer to genes in different species that have evolved from a common ancestral gene (*i.e.*, a homologous gene) by speciation. Typically, orthologs retain the same function in during the course of evolution. Identification of orthologs finds use in the reliable prediction of gene function in newly sequenced genomes.

As used herein, "paralog" and "paralogous genes" refer to genes that are related by duplication within a genome. While orthologs retain the same function through the course of evolution, paralogs evolve new functions, even though some functions are often related to the original one. Examples of paralogous genes include, but are not limited to genes encoding trypsin, chymotrypsin, elastase, and thrombin, which are all serine proteinases and occur together within the same species.

The degree of homology between sequences may be determined using any suitable method known in the art (*See e.g.*, Smith and Waterman, Adv. Appl. Math., 2:482 [1981]; Needleman and Wunsch, J. Mol. Biol., 48:443 [1970]; Pearson and Lipman, Proc. Natl. Acad. Sci. USA 85:2444 [1988]; programs such as GAP, BESTFIT, FASTA, and TFASTA in the Wisconsin Genetics Software Package (Genetics Computer Group, Madison, WI); and Devereux et al., Nucl. Acid Res., 12:387-395 [1984]). For example, PILEUP is a useful program to determine sequence homology levels. PILEUP creates a multiple sequence alignment from a group of related sequences using progressive, pairwise alignments. It can also plot a tree showing the clustering relationships used to create the alignment. PILEUP uses a simplification of the progressive alignment method of Feng and Doolittle, (Feng and Doolittle, J. Mol. Evol., 35:351-360 [1987]). The method is similar to that described by Higgins and Sharp (Higgins and Sharp, CABIOS 5:151-153 [1989]). Useful PILEUP parameters including a default gap weight of 3.00, a default gap length weight of 0.10, and weighted end gaps. Another example of a useful algorithm is the BLAST algorithm, described by Altschul *et al.,* (Altschul et al., J. Mol. Biol., 215:403-410, [1990]; and Karlin et al., Proc. Natl. Acad. Sci. USA 90:5873-5787 [1993]). One particularly useful BLAST program is the WU-BLAST-2 program (*See*, Altschul et al., Meth. Enzymol.,, 266:460-480 [1996]). parameters "W," "T," and "X" determine the sensitivity and speed of the alignment. The BLAST program uses as defaults a wordlength (W) of 11, the BLOSUM62 scoring matrix (*See*, Henikoff and Henikoff, Proc. Natl. Acad. Sci. USA 89:10915 [1989]) alignments (B) of 50, expectation (E) of 10, M'5, N'-4, and a comparison of both strands.

As used herein, "percent (%) nucleic acid sequence identity" is defined as the percentage of nucleotide residues in a candidate sequence that are identical with the nucleotide residues of the sequence.

As used herein, the term "hybridization" refers to the process by which a strand of nucleic acid joins with a complementary strand through base pairing, as known in the art.

As used herein, "maximum stringency" refers to the level of hybridization that typically occurs at about Tm-5°C (5°C below the Tm of the probe); "high stringency" at about 5°C to 10°C below Tm; "intermediate stringency" at about 10°C to 20°C below Tm; and "low stringency" at about 20°C to 25°C below Tm. As will be understood by those of skill in the art, a maximum stringency hybridization can be used to identify or detect identical polynucleotide sequences while an intermediate or low stringency hybridization can be used to identify or detect polynucleotide sequence homologs.

The phrases "substantially similar and "substantially identical" in the context of two nucleic acids or polypeptides typically means that a polynucleotide or polypeptide comprises a sequence that has at least 75% sequence identity, preferably at least 80%, more preferably at least 90%, still more preferably 95%, most preferably 97%, sometimes as much as 98% and 99% sequence identity, compared to the reference (*i.e.*, wild-type) sequence. Sequence identity may be determined using known programs such as BLAST, ALIGN, and CLUSTAL using standard parameters. (*See e.g.,* Altschul, et al., J. Mol. Biol. 215:403-410 [1990]; Henikoff et al., Proc. Natl Acad Sci. USA 89:10915 [1989]; Karin et al., Proc. Natl Acad. Sci USA 90:5873 [1993]; and Higgins et al., Gene 73:237 - 244 [1988]). Software for performing BLAST analyses is publicly available through the National Center for Biotechnology Information. Also, databases may be searched using FASTA (Pearson et al., Proc. Natl. Acad. Sci. USA 85:2444-2448 [1988]).

In some embodiments, "equivalent residues" are defined by determining homology at the level of tertiary structure for a precursor protein (*i.e.*, protein of interest) whose tertiary structure has been determined by x-ray crystallography. Equivalent residues are defined as those for which the atomic coordinates of two or more of the main chain atoms of a particular amino acid residue of the precursor protein and another protein are within 0.13nm and preferably 0.1 nm after alignment. Alignment is achieved after the best model has been oriented and positioned to give the maximum overlap of atomic coordinates of non-hydrogen protein atoms of the protein. In most embodiments, the best model is the crystallographic model giving the lowest R factor for experimental diffraction data at the highest resolution available.

In some embodiments, modification is preferably made to the "precursor DNA sequence" which encodes the amino acid sequence of the precursor enzyme, but can be by the manipulation of the precursor protein. In the case of residues which are not conserved, the replacement of one or more amino acids is limited to substitutions which produce a variant which has an amino acid sequence that does not correspond to one found in nature. In the case of conserved residues, such replacements should not result in a naturally-occurring sequence. Derivatives provided by the present invention further include chemical modification(s) that change the characteristics of the cutinase.

In some preferred embodiments, the protein gene is ligated into an appropriate expression plasmid. The cloned protein gene is then used to transform or transfect a host cell in order to express the protein gene. This plasmid may replicate in hosts in the sense that it contains the well-known elements necessary for plasmid replication or the plasmid may be designed to integrate into the host chromosome. The necessary elements are provided for efficient gene expression (*e.g.*, a promoter operably linked to the gene of interest). In some embodiments, these necessary elements are supplied as the gene's own homologous promoter if it is recognized, (*i.e.*, transcribed, by the host), a transcription terminator (a polyadenylation region for eukaryotic host cells) which is exogenous or is supplied by the endogenous terminator region of the protein gene. In some embodiments, a selection gene such as an antibiotic resistance gene that enables continuous cultural maintenance of plasmid-infected host cells by growth in antimicrobial-containing media is also included.

As used herein, "host cell" refers to a cell that has the capacity to act as a host and expression vehicle for an incoming sequence. In one embodiment, the host cell is a microorganism.

As used herein, the terms "DNA construct" and "transforming DNA" are used interchangeably to refer to DNA used to introduce sequences into a host cell or organism. The DNA may be generated *in vitro* by PCR or any other suitable technique(s) known to those in the art. In particularly preferred embodiments, the DNA construct comprises a sequence of interest (*e.g.*, as an incoming sequence). In some embodiments, the sequence is operably linked to additional elements such as control elements (*e.g.*, promoters, etc.). The DNA construct may further comprise a selectable marker. It may further comprise an incoming sequence flanked by homology boxes. In a further embodiment, the transforming DNA comprises other non-homologous sequences, added to the ends (*e.g.*, stuffer sequences or flanks). In some embodiments, the ends of the incoming sequence are closed such that the transforming DNA forms a closed circle. The transforming sequences may be wild-type, mutant or modified. In some embodiments, the DNA construct comprises sequences homologous to the host cell chromosome. In other embodiments, the DNA construct comprises non-homologous sequences. Once the DNA construct is assembled *in vitro* it may be used to: 1) insert heterologous sequences into a desired target sequence of a host cell, and/or 2) mutagenize a region of the host cell chromosome (*i.e.*, replace an endogenous sequence with a heterologous sequence), 3) delete target genes; and/or introduce a replicating plasmid into the host.

In one embodiment, the present invention involves assembling a DNA construct *in vitro,* followed by cloning of such construct into a host cell. For example, PCR fusion and/or ligation can be employed to assemble a DNA construct *in vitro.* In some embodiments, the DNA construct comprises a DNA into which a mutation has been introduced.

An "incoming sequence" as used herein means a DNA sequence that is newly introduced into the host cell. In some embodiments, the incoming sequence becomes integrated into the host chromosome or genome. The sequence may encode one or more proteins of interest. Thus, as used herein, the term "sequence of interest" refers to an incoming sequence or a sequence to be generated by the host cell. The terms "gene of interest" and "sequence of interest" are used interchangeably herein. The incoming sequence may comprise a promoter operably linked to a sequence of interest. An incoming sequence comprises a sequence that may or may not already present in the genome of the cell to be transformed (*i.e.*, homologous and heterologous sequences find use with the present invention).

As used herein, the terms "hybrid cutinases" and "fusion cutinases " refer to proteins that are engineered from at least two different or "parental" proteins. In preferred embodiments, these parental proteins are homologs of one another. For example, in some embodiments, a preferred hybrid cutinases or fusion cutinases protein contains the N-terminus of a protein and the C-terminus of a homolog of the protein. In some preferred embodiment, the two terminal ends are combined to correspond to the full-length active protein.

The term "sample" as used herein is used in its broadest sense. However, in preferred embodiments, the term is used in reference to a sample (*e.g.*, an aliquot) that comprises a variant (*e.g.*, a variant of a protein of interest) that is being analyzed, identified, modified, and/or compared with other variants and/or the wild-type protein.

As used herein, "mutations" refer to insertions, deletions, duplications, or alterations to peptide sequences or nucleic acid sequences (*e.g.*, wild-type sequences), including, but not limited to, point mutations.

As used herein, "cooperative mutations" refers to two or more amino acid mutations. In some preferred embodiments, these mutations function together in a complementary means.

As used herein, "cutinase" refers to a lipolytic enzyme capable of hydrolyzing cutin substrates. It is intended that cutinases from any source be encompassed within this definition. For example, cutinases are known to be produced by various fungi and bacterial species. Many cutinases have been described (*See e.g.*, Kolattukudy, in Borgstrom and Brockman (eds.), Lipases, Elsevier, at pages 471-504 [1984]; Longhi et al., J. Mol. Biol., 268:779-799 [1997]; U.S Pat. No. 5,827,719; WO 94/14963; WO 94/14964; WO 00/05389; Appl. Environ. Microbiol., 64: 2794-2799 [1998]; Proteins: Structure, Function and Genetics 26:442-458 [1996]; J. Comput. Chem., 17:1783-1803 [1996]; Protein Engineer., 6: 157-165 [1993]). In some embodiments, the "parent" cutinase (*i.e.*, the cutinases from which variant cutinases are designed and provided) has a nucleotide and/or amino acid sequence that is at least 50% homologous to nucleic acid and/or amino acid sequence of the cutinases of *Pseudomonas mendocina* cutinase SEQ ID NOS: 1 and SEQ ID NO: 2. In some embodiments, the parent cutinases is 60%, 70%, 80%, 90%, or greater than 90% homologous to the *Pseudomonas mendocina* cutinase nucleotide and/or amino acid sequences set forth in SEQ ID NO:1 and SEQ ID NO:2).

In some embodiments, the cutinase is naturally occurring, while in other embodiments, it is a genetically modified cutinases. In some embodiments, the genetically modified cutinases is obtained by UV irradiation, N-methyl-N'-nitrosoguanidine (NTG) treatment, ethyl methanesulfonate (EMS) treatment, nitrous acid treatment, acridine treatment or the like. In further embodiments, the modified cutinase is a recombinant cutinases produced by genetic engineering procedures such as cell fusion, gene recombination, and/or other methods known in the art.

As used herein, "grading" refers to the ranking, rating, and categorization of protein properties, including but not limited to identifying the properties as beneficial, neutral, and detrimental, and including grouping such ranked properties and marking the groups by color-coding the data and three-dimensional renditions, and including identifying structural-functional trend analysis.

As used herein, "molecular library" refers to a vector-based assembly of at least one nucleic acid molecule that is vector based, non-vector based, or combined vector and non-vector based.

As used herein, "polyester" refers to aliphilic and aromatic linear polymeric molecules containing in-chain ester groups and which are derived from the condensation of a diacid with a diol or from the polymerization of hydroxyl acids. The principal polyesters in industrial usage include polyethylene terephthalate (PET), tetramethylene terephthalate (PTMT), polybutylene terphthalate (PBT), polytrimethylene terephthalate (PTT), polylactic acid (PLA), and polyethylene naphthalate (PEN), polycyclohexanedimethylene terephthalate (CHDMT), poly(ethylene-4-oxybenzoate)A-Tell, polyglycolide, PHBA and 2GN.

As used herein, "polyesterase" refers to an enzyme that has significant capability to catalyze the hydrolysis and/or surface modification of polyester. Suitable polyesterases are isolated from animal, plant, fungal and bacterial sources. In some embodiments, the enzyme is obtained from any mutant strain (*e.g.*, obtained by UV irradiation, N-methyl-N'-nitrosoguanidine (NTG) treatment, ethyl methanesulfonate (EMS) treatment, nitrous acid treatment, acridine treatment or the like), as well as recombinant strains induced by the genetic engineering procedures such as cell fusion and gene recombination, etc.

## DETAILED DESCRIPTION OF THE INVENTION

The present application describes mutagenesis methods for protein engineering., In particular, the present application describes mutagenesis methods that include feedback adjustment from systematic result evaluation. More specifically, the, application describes site-saturation mutagenesis methods that screen for variants with one or more desirable protein properties and evaluates screening results to provide feedback for repeat screening and construction of new libraries. The invention provides cutinase variants that provide improved stability and activity compared with the wild type cutinase.

The application describes high throughput mutagenesis screening methods to select sites for saturation scanning using protein structural considerations. Site-saturation libraries are created and screened using assays for sites having protein properties of interest. The sites are categorized for the properties of interest and trends, if any, are identified. Then, at least one variant having a desired property is selected for additional library creation procedures. The additional libraries are created using feedback from the determined categories. One set of additional libraries repeats construction of the previous libraries using the feedback. A second set of additional libraries are new libraries created at sites that are expected to be beneficial based on the feedback. In some embodiments, the categories are coded, using color or other indicia, to allow easy identification of trends.

The methods utilized in the present application are described below. This description is divided into the four sections for clarity of presentation and is not meant to be limiting.

### Site-Saturation Mutagenesis

As illustrated in Figure 1, in some preferred embodiments, the site-saturation mutagenesis method is limited to a selected subset of amino acid positions in the protein defined after a thorough analysis of the protein's structure and function.

Following selection of the subset of amino acid positions in the protein for mutagenesis (*i.e.*, step 2 in Figure 1), site-saturation libraries are made at the selected sites by random substitutions of all 19 amino acids using conventional oligonucleotide-directed mutagenesis (*i.e.*, step 4 in Figure 1). During the development of the present invention, the Stratagene Quik-Change™ kit was used. However; it is not intended that the method be limited to any particular methods as conventional library production is well-known to those skilled in the art (for example, see also, Airaksinen and Hovi, Nucl. Acids Res., 26:576-581 [1998]; and the Sculptor Mutagenesis Kit (Amersham, UK)).

The mutagenic oligonucleotide employed is complementary to the areas contiguous to the selected sites. The codon for the selected sites is replaced by the sequence N,N, (G/C mixture). Because the mutagenesis method is not limited to mutations produced by a single codon substitution, the method determines site importance relative to desired protein properties, and identifies the amino-acid substitutions that produce the most efficacious results. The method is applicable to a variety of mutagenesis methods.

**Activity Screening**

Following site-saturation mutagenesis, members of the library undergo screening for the protein properties of interest using appropriate assays for the protein of interest (*i.e.*, step 6 in Figure 1). The screening preferably is conducted for more than one property to provide additional information regarding the quality and mutational effects of the screening data. The probability, "P," that a collection of variants contains at least one clone with a given amino acid is determined from the binomial distribution formula.

$$P=1-(1-p)^n$$

p= probability of finding the particular amino acid in the clone mixture
n= the number of clones screened.
For example, if 90 clones are screened, there is a 94% probability of identifying an amino acid coded for by one of the 32 possible codons.

**Systematic Result Evaluation**

Next, the activity screening results for each variant are evaluated and categorized relative to the position of the mutation in the protein to identify any structure-based trends (*i.e.*, step 8 in Figure 1). Evaluations for trends include, but are not limited to, one or more of the following examples using the assay results: (1) plotting the spatial distribution of variant sites within a three-dimensional rendition of the structure of the protein; (2) plotting the identity of the amino acid mutation; (3) plotting the distribution of beneficial mutation sites compared to amino acid sequences of similar proteins; and/or (4) grading and categorizing results, for example, as beneficial, detrimental, and neutral. The evaluated results, particularly categories, may be color-coded. The color-coding may be added to plotted data and superimposed on known, three-dimensional structural renditions of the protein. Such an analysis produces data that clearly reveal non-uniform site distribution for the various categories, and quickly points out trends for beneficial, detrimental and neutral amino acid substitutions. The analysis and result evaluation feedback enables informed selection of further mutagenesis sites and construction of new libraries.

**New Library Construction/Repeat Mutagenesis**

The data from the systematic result evaluation are used as feedback to construct new libraries and to select promising mutation sites for the previously tested libraries (*i.e.*, steps 12, 14 in Figure 1). These new libraries are constructed using the optimum variants identified as having beneficial mutations following primary mutagenesis. The optimum variants are used as templates for an additional mutagenesis procedure (*i.e.*, step 10 in Figure 1). In this construction method, libraries are made at each of the sites that produced improved or beneficial variants in the previous mutagenesis and screening steps. Following a second site-saturation mutagenesis procedure using as the parent DNA a clone with an amino acid mutation identified as beneficial, the optimum variants each have two beneficial mutations. The procedure is repeated until any protein improvement goal is reached, or until the number of beneficial sites is exhausted. This new library construction essentially repeats previous libraries using an improved starting protein chosen from one of the previous libraries and using identified beneficial mutations.
A second method of constructing a new library utilizes the trend analysis described above to mutate additional sites located near any observed and categorized beneficial mutation sites (*i.e.*, step 12 in Figure 1). This step also extends to producing mutations based on trends other than spatial proximity observed in beneficial sites. This library construction extends to new, potentially beneficial amino acid sites, and allows exclusion of categorized detrimental sites. In preferred embodiments, this construction also leads to additional improvements.

**Cutinase Variants**

In order to exemplify the utility of the present invention, numerous variant cutinases were produced using the methods described herein.
The present invention, provides cutinase variants with improved activity and/or stability compared with the wild type cutinases. In some embodiments, the cutinase variant comprises one or more amino acid substitutions at residue positions corresponding to sites 34, 73, 75, 77, 78, 79, 80, 99, 164, 191-196, 219, 223 and 225 of *Pseudomonas*

*mendocina* cutinase SEQ ID NO: 2.

A preferred cutinase variant is a variant of *Pseudomonas mendocina* cutinase SEQ ID NO: 2, wherein the variant comprises the substitutions of Phe 194 with Pro, Ser 219 with Gly, and one or more additional substitutions at amino acid positions including but not limited to 54, 78, 81, 191, 196, 200, 204, 263. For example, in some preferred embodiments, the amino acid position 54 is substituted with Ser, Glu, or Thr; the amino acid position 78 is substituted with Val; the amino acid position 81 is substituted with Ser; amino acid position 191 is substituted with Asn or His; the amino acid position 196 is substituted with Leu or Ala; the amino acid position 200 is substituted with Leu; the amino acid position 204 is substituted with Leu; the amino acid position 263 is substituted with Pro. Thus, in some embodiments, more than one amino acid is substituted in the cutinase variants as compared to the wild-type cutinase. For example, in some preferred embodiments, there are mutations at two, three, four, five, six, or more amino acid residues. In some embodiments, the variants were derived from a parent variant (*e.g.*, F180P-S205G, referred to in the Tables below). Thus, in some embodiments, multiple amino acids are substituted so as to provide additional benefits to the variant cutinases.

## EXPERIMENTAL

The following examples are provided in order to demonstrate and further illustrate certain preferred embodiments and aspects of the present invention and are not to be construed as limiting the scope thereof. These Examples describe the application of the methods to produce an enzyme having increased stability and increased hydrolytic activity. However, it is not intended that the present invention be limited to these specific enzymes.

In the experimental disclosure which follows, the following abbreviations apply: °C (degrees Centigrade); rpm (revolutions per minute); $H_2O$ (water); $dH_2O$ (deionized water); (HCl (hydrochloric acid); aa (amino acid); bp (base pair); kb (kilobase pair); kD (kilodaltons); gm (grams); μg (micrograms); mg (milligrams); ng (nanograms); μl (microliters); ml (milliliters); mm (millimeters); nm (nanometers); μm (micrometer); M (molar); mM (millimolar);, μM (micromolar); U (units); V (volts); MW (molecular weight); sec (seconds); min(s) (minute/minutes); hr(s) (hour/hours): $MgCl_2$ (magnesium chloride); NaCl (sodium chloride); $OD_{280}$ (optical density at 280 nm); $OD_{600}$ (optical density at 600 nm); PAGE (polyacrylamide gel electrophoresis); PBS (phosphate buffered saline [150 mM NaCl, 10 mM sodium phosphate buffer, pH 7.2]); MOPS (3-(N-morpholino)propanesulfonic acid ); TCA (trichloro-acetic acid); PEG (polyethylene glycol); PCR (polymerase chain reaction); RT-PCR (reverse transcription PCR); SDS (sodium dodecyl sulfate); Tris (tris(hydroxymethyl)aminomethane); w/v (weight to volume); v/v (volume to volume); LA medium (per liter: Difco Tryptone Peptone 20g, Difco Yeast Extract 10g, EM Science NaCl 1g, EM Science Agar 17.5g, dH20 to 1L): ATCC (American Type Culture Collection, Rockville, MD); Clontech (CLONTECH Laboratories, Palo Alto, CA); Difco (Difco Laboratories, Detroit, MI); GIBCO BRL or Gibco BRL (Life Technologies, Inc., Gaithersburg, MD); Invitrogen (Invitrogen Corp:, San Diego, CA); NEB (New England Biolabs, Beverly, MA); Sigma (Sigma Chemical Co., St. Louis, MO); Takara (Takara Bio Inc. Otsu, Japan); Roche (Hoffmann-La Roche, Basel, Switzerland); Testfabrics (Testfabrics, Inc., West Pittston, PA); Scientific Polymer (Scientific Polymer Products, Ontario, NY); V&P Scientific (V&P Scientific, San Diego, CA); Marsh (Marsh BioProducts, Rochester, NY); EM Science (EM Science, Gibbstown, NJ); Qiagen (Qiagen; Inc., Valencia, CA); and Stratagene (Stratagene Cloning Systems, La Jolla, CA).

## EXAMPLE 1

### Site-Saturation Mutagenesis of *Pseudomonas mendocina* Cutinase

Cutinase from *P. mendocina* (*See*, Figures 17 and 18, SEQ ID NO: 1 and SEQ ID NO:2) was subjected to methods to identify mutants that improve hydrolytic activity on polyester in poly(ethylene terephthalate) fibers and resin and/or enzyme stability.

The culture media used was buffered by MOPS and also contained 5 μg/mL chloramphenicol, to select for transformants harboring the cutinase gene. All chemicals used in these Examples were obtained from commercial vendors. The culture medium was prepared as follows:

| MOPS 1A Starter Medium - to prepare 1000 mL (filter sterilize) | |
|---|---|
| 100 mL | 10x MOPS (see below) |
| 12.6 g | Glucose |
| 0.23 g | $K_2HPO_4$ |
| 0.53 g | $NH_4Cl$ |
| | Adjust pH to 7.3 with KOH |

**MOPS-Urea Medium - to prepare 1000 mL (filter sterilize)**

| | |
|---|---|
| 100 mL | 10x MOPS (see below) |
| 0.52 g | $K_2HPO_4$ |
| 3.6 g | Urea |
| 21 g | Glucose |
| 35 g | Maltrin-150 |
| | Adjust pH to 7.4 with KOH |

**10x MOPS - to prepare 1000 mL (filter sterilize)**

| | |
|---|---|
| 83.7 g | MOPS |
| 7.2 g | Tricine |
| 12.0 g | KOH |
| 0.48 g | $K_2SO_4$ |
| 1.07 g | $MgCl_2.6H_2O$ |
| 29.2 g | NaCl |
| 100 mL | 100x Micronutrients (see below) |

**100x Micronutrients - to prepare 1000 mL (filter sterilize)**

| | |
|---|---|
| 400 mg | $FeSO_4.7H_2O$ |
| 100 mg | $MnSO_4.H_2O$ |
| 100 mg | $ZnSO_4.7H_2O$ |
| 50 mg | $CuCl_2.2H_2O$ |
| 100 mg | $CoCl_2.6H_2O$ |
| 100 mg | $NaMoO_4.2H_2O$ |
| 100 mg | $Na_2B_4O_7.10H_2O$ |
| 1.1g | $CaCl_2$ (or 10 mL of 1.0 M stock) |
| 1.47 g | Sodium citrate |

**I. Primary Screening Site-Saturation Mutagenesis**

Site-saturation variant libraries were created at amino acid positions corresponding to residue positions 71-80, 82, 99, 100, 102, 139, 141, 144, 162-166, 168, 169, 190-197 and 218-225 of SEQ ID NO:2 in *P. mendocina* cutinase, expressed in *Bacillus subtilis* strain 3934. The nucleic acid sequence of the parent cutinase is attached hereto as Figure 17 (SEQ ID NO:1). Libraries were created using the Stratagene Quik-Change™ kit using oligonucleotide primers randomized with NN(G/C) at the target position. Each selected amino acid was randomly replaced with all 19 possible alternatives. Colonies obtained from the variant libraries were selected and placed into 96-well growth plates that contained 100 μL/well of MOPS 1A starter medium. The plates were incubated at 37°C overnight with continuous humidified shaking at 260 rpm. A sample from each well was replicated into a Millipore 96-well filter plate that contained 200 μL/well of MOPS-Urea medium. Then, 50 μL of 45% glycerol was added to each well of the initial growth plates, which then were stored at -70°C.

The filter plates were incubated for 4 days at 37° C with continuous humidified shaking at 260 rpm, to allow growth and enzyme production. Next, the plates were filtered and assayed for hydrolysis activity using a polyester hydrolysis assay. In addition, the extracellular cutinase concentration was determined using a TCA turbidity assay, and the thermal stability in laundry detergent was also assessed.

**II. Assays**

The assays used to characterize the variant enzymes included the following assay systems.

### A. Assay for Hydrolysis Activity on Polyester:

This polyester hydrolysis assay monitors the release of soluble terephthalate-containing fragments resulting from the enzymatic hydrolysis of insoluble polyester (polyethylene terephthalate or PET). The fragments have a significant absorbance at approximately 240-250 nm.

Insoluble PET polyester substrates in the form of fabric samples (Dacron 54 from Testfabrics) or PET resin powder (Scientific Polymer Products catalog # 138, cryogenically ground to < 400 μm particle size) were used. The powdered PET samples were placed into a dispensing device (V&P Scientific), to distribute approximately 18 mg uniform samples into the wells of 96-well conventional assay plates. When PET fabric samples were used, 5/8-inch circular pieces of the fabric were cut and placed into 12-well assay plates.

For the PET powder hydrolysis assay, two plates were prepared, one containing PET powder and one with no substrate. Each of the 96 wells of the two assay plates received 200 μL of a reaction buffer containing 50 mM Tris pH 8.6 and 0.01% Brij-35 (Sigma #430AG-6). The plate without PET substrate was used as an assay blank.

Twenty μl of each enzyme variant samples were added to appropriate wells in each of the two assay plates. Both assay plates were sealed with plate sealers (Marsh). The assay plate containing the PET sample was placed in a covered 40°C incubator, and the plate was incubated for 18 hours with shaking at 250 rpm. The covered blank assay plate was maintained at room temperature.

Following incubation, 180 μL samples from each well of the PET substrate plate were transferred to a filter plate with a 12-channel pipettor device. The ends of the pipettes were removed by cutting to avoid clogging with the PET powder. Each sample of the PET filter plate was filtered into a well of a new 96-well assay plate.

Then, 100 μl from each well of the substrate plate and the blank plate were transferred to a UV-transparent plate, ahd the absorbance of each sample was determined in a plate reader at 250 nm. The absorbance of the wells from the blank plate without substrate was subtracted from the corresponding wells in the assay plate with PET substrate.

### B. TCA Assay to Determine Cutinase Concentration

Twenty μL of the filtered culture supernatant were added to each well of a 96-well flat bottom plate which contained 100 μL/well of purified water. Light scattering/absorbance was determined at 410 nm using 5 second mixing mode in a plate reader to determine base-line blank values. Then, 100 μL of 15% w/v TCA were added to each well prior to incubating the plate for between 5 and 30 minutes at room temperature. Light scattering/absorbance caused by protein precipitation was determined at 410 nm using a plate reader set for 5 second mixing mode.

The results were calculated by subtracting the blank reading, which did not contain TCA, from the sample readings with TCA. The light scattering/absorbance results were determined to be linear with respect to the enzyme concentrations present in the samples, thereby allowing direct plotting of concentration against enzyme performance as shown in Figure 2. Figure 2 shows the results obtained from the untransformed host strain (PE-1), three wild-type cutinase transformants (PE-2 through PE-4) and one negative control, transformed with a cellulase gene (BCE). Good correlation is observed between polyester hydrolysis activity and the TCA signal. These graphs help facilitate identification and selection of variants with elevated hydrolytic activity.

Figures 3 and 4 provide data from two site-saturation libraries involving amino acid positions Leu 168| (Library A) and Phe 194 (Library B). As indicated, the wild type controls fall along the same activity-concentration line; the line shown is the best fit to these data points calculated using linear regression. The graphs also show that the variants fall above, below, and along this line, signifying better, worse, or similar specific activity relative to the wild-type controls. In addition, as indicated by the graphs, the library at position 194 exhibits more diversity and more improved variants than the library at position 168.

### C. Enzyme Stability Assays

Stability assays were conducted to determine thermostability of the variants in a detergent medium. The stability of the variants obtained from the site-saturation mutagenesis procedure was determined by adding 10 μL of the filter plate samples into each well of two 96-well plates containing 200 μL/well of liquid detergent (1.6 g/L, 6.3 gpg hardness). The 2 plates were mixed, and one plate was maintained on ice or in an ice-water bath (unstressed plate) while the second plate was incubated in a 40° C water bath for 10 minutes. (thermostressed plate). The stressed plate was transferred to the ice-water bath, and esterase activity assays were conducted on both plates as described below. Wild type cutinase samples also were run as above for comparison with the variants.

The enzyme activity assay used in these experiments is based upon the cleavage of colorless p-nitrophenyl butyrate (pNB) with esterase in the presence of water to produce butyrate and yellow colored p-nitrophenolate. The assay was conducted using an assay buffer containing 100 mM Tris pH 8.0 and 0.1% (v/v) Triton X-100. The substrate stock was 100 mM p-nitrophenyl butyrate in DMSO, prepared by adding 174.3 μL pNB to 10 mL DMSO. The substrate stock was divided into 1-mL aliquots and stored at -20° C until use.

The pNB assay procedure was as follows: substrate stock (400 μL) was diluted into 40 mL assay buffer for each pair of assay plates. Then, 200 μL of this diluted substrate solution were added to each well of conventional 96-well plates. The plates were equilibrated at 25° C, and the reaction was initiated by adding 10 μL of the enzyme/detergent solution from a stressed or unstressed plate. The plate was mixed and inserted into the plate reader and data acquisition was begun. The activity rate ($\Delta mAU_{410}$/minute) was determined. The pNB reaction rate is proportional to the concentration of active enzyme. For each well, the fraction of active enzyme after stress was calculated using the following formula:

Fraction active=(stressed pNB activity-blank)/(unstressed pNB activity -blank).

The fraction of active enzyme after stress was plotted against the pNB activity of the unstressed enzyme for each variant, as shown in Figures 5 and 6, which depict the results for site-saturation libraries at amino acid positions Leu 168 (Library A) and Phe 194 Library B). The results shown in Figures 5 and 6 show thermostable variants having unstressed pNB activity, and also identify some variants which have apparently elevated thermostability only because the pNB activity is too low to produce an accurate ratio. Figures 5 and 6 further demonstrate that mutations in different sites produce different enzymatic properties. For instance, the library shown in Figure 5 produced only a few promising variants with activity greater than wild-type, while the library shown in Figure 6 produced a number of variants with considerably greater activity than the wild-type enzyme.

**D. Systematic Result Evaluations**

The results from the TCA assay, the PET hydrolysis assay, and the stability assay were analyzed using conventional, known formulas as set out above, using Excel™ software. Variants were graded and ranked for stability and hydrolysis activity. Table 1 shows the results obtained from the site-saturation mutagenesis procedure described in this Example. The results were graded and color coded as follows: green=more than 2% of the variants at the listed sites had significantly greater activity than the control (beneficial mutation); blue=the majority of the variants had unchanged activity (neutral mutation); red=the majority of the variants had significantly decreased activity relative to the control (detrimental mutation) ; and brown=approximately half of the variants had unchanged activity and half had decreased activity (neutral mutation). The color-coding was repeated using the same colors and same percentage criteria for stability results. As indicated in Table 1, color coding easily identifies those variants having both desired enzyme properties.

## Table 1

| Site | wt Res. | # Clones | Specific PETase Activity % Up | % Neutral | % Down | Stability % Up | % Neutral | % Down |
|---|---|---|---|---|---|---|---|---|
| 71 | Gly | 135 | 0.0 | 8.1 | 91.9 | 0.0 | 28.9 | 71.1 |
| 72 | Thr | 168 | 1.2 | 67.3 | 31.5 | 0.0 | 53.6 | 46.4 |
| 73 | Gly | 131 | 0.8 | 61.8 | 37.4 | 9.9 | 74.0 | 16.0 |
| 74 | Ala | 179 | 1.7 | 77.1 | 21.2 | 0.0 | 76.0 | 24.0 |
| 75 | Gly | 132 | 0.0 | 75.0 | 25.0 | 0.0 | 81.1 | 18.9 |
| 76 | Pro | 100 | 0.0 | 48.0 | 52.0 | 0.0 | 68.0 | 32.0 |
| 77 | Ser | 159 | 11.9 | 77.4 | 10.7 | 1.3 | 97.5 | 1.3 |
| 78 | Thr | 178 | 2.2 | 77.5 | 20.2 | 5.1 | 75.3 | 19.7 |
| 79 | Tyr | 179 | 4.5 | 43.0 | 52.5 | 0.0 | 67.0 | 33.0 |
| 80 | Ala | 138 | 0.7 | 82.6 | 16.7 | 0.7 | 92.8 | 6.5 |
| 82 | Leu | 134 | 0.0 | 27.6 | 72.4 | 0.0 | 50.7 | 49.3 |
| 99 | Ser | 133 | 1.5 | 78.2 | 20.3 | 0.0 | 87.2 | 12.8 |
| 100 | Asn | 174 | 1.1 | 47.7 | 51.1 | 0.6 | 97.7 | 1.7 |
| 102 | Gly | 145 | 0.0 | 4.8 | 95.2 | 0.0 | 33.8 | 66.2 |
| 139 | His | 110 | 0.9 | 10.9 | 88.2 | 0.0 | 45.5 | 54.5 |
| 140 | Ser | 158 | 0.0 | 9.5 | 90.5 | 0.0 | 13.3 | 86.7 |
| 141 | Gln | 179 | 0.6 | 38.0 | 61.5 | 0.0 | 39.7 | 60.3 |
| 144 | Gly | 166 | 1.2 | 25.9 | 72.9 | 0.0 | 48.2 | 51.8 |
| 162 | Gln | 180 | 1.1 | 38.3 | 60.6 | 0.0 | 27.8 | 72.2 |
| 163 | Pro | 179 | 0.0 | 2.8 | 97.2 | 0.0 | 34.1 | 65.9 |
| 164 | Tyr | 179 | 1.7 | 30.2 | 68.2 | 4.5 | 37.4 | 58.1 |
| 165 | Thr | 167 | 1.8 | 28.7 | 69.5 | 0.0 | 69.5 | 30.5 |
| 166 | Leu | 180 | 1.1 | 67.2 | 31.7 | 0.0 | 78.3 | 21.7 |
| 168 | Leu | 180 | 0.6 | 67.8 | 31.7 | 0.6 | 70.6 | 28.9 |
| 169 | Gly | 148 | 0.7 | 57.4 | 41.9 | 0.0 | 70.9 | 29.1 |
| 190 | Asp | 180 | 0.0 | 12.8 | 87.2 | 0.0 | 45.6 | 54.4 |
| 191 | Thr | 180 | 0.6 | 47.2 | 52.2 | 6.7 | 73.3 | 20.0 |
| 192 | Ile | 179 | 3.9 | 60.9 | 35.2 | 0.0 | 54.7 | 45.3 |
| 193 | Ala | 173 | 2.3 | 16.2 | 81.5 | 0.0 | 39.3 | 60.7 |
| 194 | Phe | 173 | 8.1 | 61.8 | 30.1 | 2.3 | 81.5 | 16.2 |
| 195 | Pro | 178 | 2.8 | 34.3 | 62.9 | 0.0 | 54.5 | 45.5 |
| 196 | Tyr | 166 | 9.6 | 83.7 | 6.6 | 15.7 | 83.1 | 1.2 |
| 197 | Leu | 179 | 1.7 | 41.3 | 57.0 | 0.0 | 45.3 | 54.7 |
| 218 | Val | 147 | 0.0 | 21.1 | 78.9 | 0.0 | 28.6 | 71.4 |
| 219 | Ser | 179 | 2.8 | 69.8 | 27.4 | 2.2 | 74.9 | 22.9 |
| 220 | His | 160 | 0.6 | 3.8 | 95.6 | 0.0 | 26.9 | 73.1 |
| 221 | Phe | 177 | 1.1 | 61.0 | 37.9 | 0.0 | 48.0 | 52.0 |
| 222 | Glu | 179 | 0.0 | 1.1 | 98.9 | 0.0 | 5.0 | 95.0 |
| 223 | Pro | 136 | 4.4 | 41.9 | 53.7 | 0.0 | 47.1 | 52.9 |
| 224 | Val | 180 | 1.7 | 56.7 | 41.7 | 0.6 | 75.6 | 23.9 |
| 225 | Gly | 165 | 2.4 | 33.3 | 64.2 | 3.6 | 53.3 | 43.0 |

**Table 1** shows the results obtained from a typical enzyme system. The results are color-coded as follows:
Green ( )--more than 2% of the clones had significantly greater activity than control.
Blue ( ) – the majority were unchanged. Red ( ) – the majority had significantly decreased activity relative to the control. Brown ( ) – approximately half were unchanged and half were decreased.

Secondary screening to repeat the previous libraries and create new libraries was conducted using the variants having beneficial properties, as shown in Table 1. The frozen glycerol stock samples for each improved variant were streaked onto appropriate media and incubated to obtain single colonies. The colonies from each variant were inoculated into tubes containing 3 mL LB medium and shaken at 37° C for 6-18 hours.

Then, 800 μL of broth from each incubated tube was combined with 400 μL of 45% glycerol and stored at -70° C. One mL of broth was centrifuged, the supernatant was removed, and the pellet was stored at -20° C for DNA sequencing.

Then, 100 μL of each broth was pipetted in multiple replicates into a 96-well plate. A sample from each well was replicated into a filter plate containing 200 μL/well of MOPS-Urea medium. The samples present on the filter plates were allowed to grow for 4 days at 37° C with humidified shaking at 260 rpm. The plates were filtered and the TCA, PET hydrolysis, and stability assays described above were conducted. The results were calculated as described above, and the samples of the highest performing variants were pooled. The enzyme concentration in the pooled sample was determined by SDS-PAGE using densitometry or LC/MS using an $^{15}N$-labeled wild-type cutinase standard. The variants were tested for activity on polyester fabric in buffer and in detergent. The results are shown in Table 2, which lists the site and substitution made for variants with elevated stability. Table 3 indicates the variants with improved hydrolysis of polyester (PET). The F180-S205 last seven variants shown in Table 2 represent a second library as created in step 14 of Figure 1 (using the S205G variant as a template), showing two beneficial mutations which greatly increased relative stability of these seven variants. A number of variants with two mutations are also indicated in Table 3.

**Table 2. Polyesterase Variants with Increased Stability (Secondary Screen)**

| Mutations in Variants | Relative Stability of Variants Compared to Wild-Type |
|---|---|
| F194I | 1.65 |
| F194L | 1.89 |
| F194N | 1.66 |
| F194P | 1.63 |
| G73F | 2.02 |
| G73K | 2.62 |
| G73L | 2.78 |
| G73V | 3.19 |
| S219G . | 2.47 |
| S34R | 1.38 |
| S77R | 2.23 |
| S99H | 2.33 |
| S99K | 2.56 |
| T191H | 2.56 |
| T191 L | 2.68 |
| T191Y | 2.68 |
| T78A | 2.26 |
| T78L | 2.63 |
| Y164F | 1.87 |
| Y196A | 228 |
| Y196L | 2.28 |
| Y196P | 3.21 |
| F194A-S219G | 8.35 |
| F194H-S219G | 13.17 |
| F194K-S219G | 10.14 |
| F194L-S219G | 8.84 |
| F194N-S219G | 11.58 |
| F194P-S219G | 10.56 |
| F194S-S219G | 7.27 |

**Table 3. Polyesterase Variants and Performance Summary (Tertiary Screen)**

| Mutations in Variants | Relative Buffer Activity of Variants Compared to Wild-Type | Relative Detergent Activity of Variants Compared to Wild-Type |
|---|---|---|
| A80E | 1.41 | 1.22 |
| F194A-S219G | 1.31 | 2.31 |
| F194H-S219G | 1.18 | 1.75 |
| F1941 | 2.21 | 2.24 |
| F194K-S219G | 101 | 2.87 |
| F194L-S219G | 1.18 | 1.95 |
| F194N | 1.77 | 1.83 |
| F194N-S219G | 1.24 | 2.02 |
| F194P-S219G | 1.64 | 3.43 |
| F194S-S219G | 1.54 | 2.62 |
| G73F | 1.13 | 1.09 |
| G73L | 1.18 | 1.30 |
| G75D | 1.85 | 2.07 |
| G75E | 1.73 | 2.16 |
| 1192M-F194V-S219G | 2.07 | 2.99 |
| R54Q-Y126Y | 1.33 | 1.10 |
| S219N-F221L | 0.82 | 1.15 |
| S77R | 0.77 | 1.12 |
| S99K | 1.18 | 1.58 |
| T191H | 1.62 | 2.55 |
| T191L | 1.50 | 2.24 |
| T191Y | 1.58 | 2.73 |
| T78L | 1.30 | 1.27 |
| Y196A | 1.28 | 1.57 |
| Y196P | 1.21 | 1.38 |

The data in Table 3 were obtained by comparing samples of the variants having the indicated mutations with wild-type cutinase. These comparisons were made using equal enzyme concentrations in the "Tertiary screen" (i.e., overnight hydrolysis of 5/8" swatches of Dacron 54 polyester fabric in 12-well plates held at 40°C). Liquid laundry detergent was used in these experiments at a concentration standard in the industry.

The improvements in the properties of the second generation variants when tested in a detergent environment are shown graphically in Figures 15 and 16. Figure 15 indicates that the second generation variant has a 3-fold increase in activity as compared to the wild type when plotted against enzyme concentration. Figure 16 shows that the thermostability of the second generation variant is greatly increased as compared to the wild type.

A summary of the results of the method of the present invention includes the initial screening procedure results shown in Table 1. Table 1 provides a list of sites where mutations produced variants having at least some polyesterase activity and some stability: Additionally, Table 1 shows that mutations at the following sites produced variants having more than two percent of the clones with greater activity than controls: sites, 77-79, 192-196, 219, 223, and 225. Mutations at the following sites resulted in variants having more than two percent of the clones with greater stability than controls include sites 73, 78, 164, 191, 194, and 196. The following sites represent variants with both improved activity and stability include sites 78, 194, 196 and 225. Clones were selected based upon the promising sites shown in Table 1, and secondary screening was conducted by repeating the previous libraries and by creating knew libraries at new sites expected to be beneficial.

Tables 2 and 3 represent, respectively, secondary stability screening steps and tertiary activity screening steps using the selected clones identified during primary screening as templates. Tables 2 and 3 identify the mutations made at the selected sites and include combinations of mutations. The substitutions of serine at locations 34 and 99 represent the creation of new libraries at new sites which are expected to be beneficial.

Table 2 shows that substitution of phenylalanine at site 194 with isoleucine, leucine, asparagines, or proline resulted in variants having improved stability as compared to the wild-type; substitution of glycine at site 73 with phenylalanine, lysine, leucine, or valine produced variants with improved stability as compared to the wild type. Substitutions of phenylalanine at site 194 in combination with substitutions of serine at site 219 resulted in variants with significantly increased relative stability.

As indicated in Table 3, specific substitutions at one to three sites produced variants with improved polyesterase activity.

### D. Structural Context Evaluation

The graded screening results (amino acid changes that resulted in improvement, detriment, or neutral) were examined in the context of position within the protein to elucidate any underlying structurally based trends. The results are shown in bold superimposed on maps of the structure of the protein, as shown in Figures 7-14. The results also were color-coded (not shown) using the same colors used in Table 1. Figure 7 shows the catalytic triad active site face (in bold) of the *Pseudomonas mendocina* cutinase. (Ser 140, Asp 190, His 220). Figure 8 shows the addition of the initial target amino acids (in bold) that were used for the site-saturation scanning mutagenesis procedure. Figure 9 shows the sites (in bold) that resulted in at least 2% of the variants having significantly improved polyester hydrolysis activity. Figure 10 shows the variant sites (in bold) in which the majority of mutations had no effect on activity. Figure 11 shows the variant sites (in bold) in which the majority of mutations generally had a detrimental effect on activity. Figure 12 shows the variant sites (in bold) which resulted in a least 2% of the variants having significantly improved stability. Figure 13 shows the variant sites (in bold) in which the majority of mutations generally had no effect on stability. Figure 14 shows the variant sites (in bold) in which the majority of mutations generally had a detrimental effect on stability

Figures 7-14 relate the variants to structure, thereby suggesting further modification sites. For instance, in the examples shown, detrimental sites tend to be buried in both the activity and stability maps and confined to generally the same locations. Improvement sites for activity and stability tend to occupy similar locations within the structure of the protein. Sites with no effect also occupy similar positions within the protein structure for both activity and stability results.

## EXAMPLE 2

### Additional Cutinase Variants

Clone number PE051-2-A2, which has mutations F194P-S219G, was subjected to additional site-saturation mutagenesis to create additional mutations according to protocols described in Example 1. Table 4 lists the site and substitution made for variants with elevated stability compared with the parent variant. Table 5 lists the site and substitution made for variants with elevated activity compared with the parent variant.

**Table 4. Polyesterase Variants with Increased Stability (Secondary Screen)**

| Parent Variant | Additional Mutations | Relative Stability of Additional Mutations Compared to Parent Variant |
|---|---|---|
| F194P-S219G | T191N | 1.59 |
| F194P-S219G | T191H | 1.73 |
| F194P-S219G | Y196L-Q200L | 1.50 |
| F194P-S219G | Y196L | 1.32 |
| F194P-S219G | Y196A | 1.35 |
| F194P-S219G | T78V | 1.28 |
| F194P-S219G | R54T-T191N | 1.23 |
| F194P-S219G | R54S-T191H | 1.34 |
| F194P-S219G | R54T-T191N | 1.87 |
| F194P-S219G | R54T-T78V-T191N-L263P | 3.15 |
| F194P-S219G | R54T-T78V-T191 N-Y196L- P204L-L262P | 3.45 |

**Table 5. Polyesterase Variants with Increased Activity (Tertiary Screen)**

| Parent Variant | Mutations | Relative Activity in Buffer of Variant Mutations Compared to Parent Variant | Relative Activity in Detergent of Variant Mutations Compared to Parent Variant |
|---|---|---|---|
| F194P-S219G | T191N | 1.20 | 0.71 |
| F194P-S219G | Y196L-Q200L | 1.37 | 0.64 |

(continued)

| Parent Variant | Mutations | Relative Activity in Buffer of Variant Mutations Compared to Parent Variant | Relative Activity in Detergent of Variant Mutations Compared to Parent Variant |
|---|---|---|---|
| F194P-S219G | Y196L | 1.27 | 0.81 |
| F194P-S219G | Y196A | 1.25 | 0.92 |
| F194P-S219G | R54S | 2.06 | 1.06 |
| F194P-S219G | R54T | 2.00 | 1.13 |
| F194P-S219G | T78V | 1.69 | 0.92 |
| F194P-S219G | R54E | 1.41 | 1.31 |
| F194P-S219G | R54T-T191N | 1.26 | 1.05 |
| F194P-S219G | R54E-Y196L | 1.81 | 1.48 |
| F194P-S219G | R54E-T191N | 1.52 | 1.27 |
| F194P-S219G | R54S-Y196A-G81S | 1.42 | 1.46 |
| F194P-S219G | R54T | 1.45 | 1.11 |
| F194P-S219G | R54T-T191N | 1.50 | 1.09 |
| F194P-S219G | R54T-T78V-T191N-L263P | 1.83 | 1.29 |
| F194P-S219G | R54T-T78V-T191N-Y196L-R204L-L263P | 2.39 | 1.61 |

The methods described herein find use in the engineering of proteins with enhanced properties for use in fields other than textile modifications as described above. Such fields include, but are not limited to, healthcare, foods and feed, brewing, cosmetics, agriculture, textiles, detergents, environmental waste conversion, biopulp processing, biomass conversion to fuel, and other chemical procedures. Indeed, the methods find use in the improvement of proteins for any application.

The examples above are not meant to be limiting, and the method is intended to apply to mutagenesis of any protein. For those proteins where three dimensional maps are not available and only the amino acid sequence is known, the method may be carried out by comparing the known sequence to other sequences which are homologous. Using this method, dissimilar sites or sites with suspected functional importance are mutated because non-conserved sites generally are more readily available to accommodate amino acid substitutions.

## Claims

1. A cutinase variant comprising a substitution of one or more amino acids at residue positions corresponding to sites 34, 73, 75, 77, 78, 79, 80, 99, 164, 191-196, 219, 223 and 225 of *Pseudomonas mendocina* cutinase SEQ ID NO: 2, wherein said cutinase variant is thermostable and has hydrolytic activity on polyester.

2. A cutinase variant of Claim 1, wherein said variant comprises the substitutions of Phe 194 with Pro, Ser 219 with Gly, and one or more additional substitutions selected from the amino acid positions 54, 78, 81, 191, 196, 200, 204, and 263.

3. The cutinase variant of Claim 2 wherein:

   (a) the amino acid position 54 is substituted with Ser, Glu, or Thr; or
   (b) the amino acid position 78 is substituted with Val; or
   (c) the amino acid position 81 is substituted with Ser; or
   (d) the amino acid position 191 is substituted with Asn or His; or
   (e) the amino acid position 196 is substituted with Leu or Ala; or
   (f) the amino acid position 200 is substituted with Leu; or
   (g) the amino acid position 204 is substituted with Leu; or
   (h) the amino acid position 263 is substituted with Pro.

**4.** A cutinase variant of Claim 1, wherein said cutinase variant has polyesterase activity.

**5.** The cutinase variant of Claim 4, wherein said cutinase variant has increased stability as compared to wild-type cutinase and comprises

(a) the substitution of Gly 73 with an amino acid selected from the group consisting of Phe, Lys, Leu, and Val; or
(b) the substitution of Thr 191 with an amino acid selected from the group consisting of His, Leu, and Tyr; or
(c) the substitution of Thr 78 with an amino acid selected from the group consisting of Ala, Leu and Val; or
(d) the substitution of Tyr 164 with Phe; or
(e) the substitution of Tyr 196 with an amino acid selected from the group consisting of Ala, Leu, and Pro; or
(f) the substitution of Phe 194 and substitution of Ser 219; or
(g) the substitution of Ala 80 with Glu.

**6.** The cutinase variant of Claim 5(f), wherein said Phe 194 is substituted with an amino acid selected from the group consisting of Ala, His, Lys, Leu, Asn, Pro, and Gly; and wherein said Ser 219 is substituted with Gly.

**7.** The cutinase variant of Claim 6, wherein said cutinase variant has increased polyesterase activity as compared to wild-type cutinase.

**8.** The cutinase variant of Claim 4, wherein said cutinase variant has increased polyesterase activity as compared to wild-type cutinase and comprises

(a) the substitution of Phe 194 with Ile; or
(b) the substitution of Phe 194 with an amino acid selected from the group consisting of Lys and Leu, and the substitution of Ser 219 with Gly; or
(c) the substitution of Phe 194 with Asn; or
(d) the substitution of Phe 194 with an amino acid selected from the group consisting of Asn, Pro, and Ser, and the substitution of Ser 219 with Gly; or
(e) the substitution of Gly 73 with an amino acid selected from the group consisting of Phe and Leu; or
(f) the substitution of Gly 75 with an amino acid selected from the group consisting of Asp and Glu; or
(g) the following substitutions Ile 192 with Met; Phe 194 with Val; and Ser 219 with Gly; or
(h) the substitution of Ser 219 with Asn, and substitution of Phe 221 with Leu; or
(i) the substitution of Ser 99 with Lys; or
(j) the substitution of Thr 191 with an amino acid selected from the group consisting of His, Leu, and Tyr; or'
(k) the substitution of Thr 78 with Leu; or
(l) the substitution of Tyr 196 with an amino acid selected from the group consisting of Ala and Pro; or
(m) the substitution of Arg 204 with Leu; or
(n) the substitution of Leu 263 with Pro.

**9.** The cutinase variant of Claim 4, wherein said cutinase variant has increased stability as compared to wild-type cutinase and comprises

(a) the substitution of Phe 194 with an amino acid selected from the group consisting of Ile, Leu, Asn, and Pro; or
(b) the substitution of Arg 204 with Leu; or
(c) the substitution of Leu 263 with Pro.

**Patentansprüche**

**1.** Cutinase-Variante, umfassend eine Substitution einer oder mehrerer Aminosäuren an Aminosäurerestpositionen, die den Stellen 34, 73, 75, 77, 78, 79, 80, 99, 164, 191-196, 219, 223 und 225 von Pseudomonas-mendocina-Cutinase (Seq.-ID Nr. 2) entsprechen, worin die Cutinase-Variante thermostabil ist und eine hydrolytische Aktivität in Bezug auf Polyester aufweist.

**2.** Cutinase-Variante nach Anspruch 1, worin die Variante Substitutionen von Phe 194 durch Pro, Ser 219 durch Gly und eine oder mehrere zusätzliche Substitutionen umfasst, die aus den Aminosäurepositionen 54, 78, 81, 191, 196, 200, 204 und 263 ausgewählt sind.

**3.** Cutinase-Variante nach Anspruch 2, worin:

(a) die Aminosäureposition 54 durch Ser, Glu oder Thr substituiert ist; oder
(b) die Aminosäureposition 78 durch Val substituiert ist; oder
(c) die Aminosäureposition 81 durch Ser substituiert ist; oder
(d) die Aminosäureposition 191 durch Asn oder His substituiert ist; oder
(e) die Aminosäureposition 196 durch Leu oder Ala substituiert ist; oder
(f) die Aminosäureposition 200 durch Leu substituiert ist; oder
(g) die Aminosäureposition 204 durch Leu substituiert ist; oder
(h) die Aminosäureposition 263 durch Pro substituiert ist.

**4.** Cutinase-Variante nach Anspruch 1, worin die Cutinase-Variante Polyesterase-Aktivität aufweist.

**5.** Cutinase-Variante nach Anspruch 4, worin die Cutinase-Variante im Vergleich zu Wildtyp-Cutinase eine höhere Stabilität aufweist und Folgendes umfasst:

(a) die Substitution von Gly 73 durch eine aus der aus Phe, Lys, Leu und Val bestehenden Gruppe ausgewählte Aminosäure; oder
(b) die Substitution von Thr 191 durch eine aus der aus His, Leu und Tyr bestehenden Gruppe ausgewählte Aminosäure; oder
(c) die Substitution Thr 78 durch eine aus der aus Ala, Leu und Val bestehenden Gruppe ausgewählte Aminosäure; oder
(d) die Substitution von Tyr 164 durch Phe; oder
(e) die Substitution von Tyr 196 durch eine aus der aus Ala, Leu und Pro bestehenden Gruppe ausgewählte Aminosäure; oder
(f) die Substitution von Phe 194 und die Substitution von Ser 219; oder
(g) die Substitution von Ala 80 durch Glu.

**6.** Cutinase-Variante nach Anspruch 5(f), worin Phe 194 durch eine aus der aus Ala, His, Lys, Leu, Asn, Pro und Gly bestehenden Gruppe ausgewählte Aminosäure substituiert ist und worin Ser 219 durch Gly substituiert ist.

**7.** Cutinase-Variante nach Anspruch 6, worin die Cutinase-Variante im Vergleich mit Wildtyp-Cutinase eine höhere Polyesterase-Aktivität aufweist.

**8.** Cutinase-Variante nach Anspruch 4, worin die Cutinase-Variante im Vergleich zu Wildtyp-Cutinase eine höhere Polyesterase-Aktivität aufweist und Folgendes umfasst:

(a) die Substitution von Phe 194 durch Ile; oder
(b) die Substitution von Phe 194 durch eine aus der aus Lys und Leu bestehenden Gruppe ausgewählte Aminosäure und die Substitution von Ser 219 durch Gly; oder
(c) die Substitution von Phe 194 durch Asn; oder
(d) die Substitution von Phe 194 durch eine aus der aus Asn, Pro und Ser bestehenden Gruppe ausgewählte Aminosäure und die Substitution von Ser 219 durch Gly; oder
(e) die Substitution von Gly 73 durch eine aus der aus Phe und Leu bestehenden Gruppe ausgewählte Aminosäure; oder
(f) die Substitution von Gly 75 durch eine aus der aus Asp und Glu bestehenden Gruppe ausgewählte Aminosäure; oder
(g) folgende Substitutionen: Ile 192 durch Met; Phe 194 durch Val und Ser 219 durch Gly; oder
(h) die Substitution von Ser 219 durch Asn und die Substitution von Phe 221 durch Leu; oder
(i) die Substitution von Ser 99 durch Lys; oder
(j) die Substitution von Thr 191 durch eine aus der aus His, Leu und Tyr bestehenden Gruppe ausgewählte Aminosäure;
(k) die Substitution von Thr 78 durch Leu; oder
(l) die Substitution von Tyr 196 durch eine aus der aus Ala und Pro bestehenden Gruppe ausgewählte Aminosäure; oder
(m) die Substitution von Arg 201 durch Leu; oder
(n) die Substitution von Leu 263 durch Pro.

**9.** Cutinase-Variante nach Anspruch 4, worin die Cutinase-Variante im Vergleich zu Wildtyp-Cutinase eine höhere Stabilität aufweist und Folgendes umfasst:

(a) die Substitution von Phe 194 durch eine aus der aus Ile, Leu, Asn und Pro bestehenden Gruppe ausgewählte Aminosäure; oder
(b) die Substitution von Arg 204 durch Leu; oder
(c) die Substitution von Leu 263 durch Pro.

**Revendications**

**1.** Variante de cutinase comprenant une substitution d'un ou de plusieurs acides aminés à des positions de résidu correspondant aux sites 34, 73, 75, 77, 78, 79, 80, 99, 164, 191-196, 219, 223 et 225 de *Pseudomonas mendocina* cutinase SEQ ID NO: 2, où ladite variante de cutinase est thermostable et possède une activité hydrolytique sur le polyester.

**2.** Variante de cutinase selon la revendication 1, où ladite variante comprend les substitutions de Phe 194 par Pro, Ser 219 par Gly, et une ou plusieurs substitutions additionnelles sélectionnées parmi les positions des acides aminés 54, 78, 81, 191, 196, 200, 204 et 263.

**3.** Variante de cutinase selon la revendication 2, dans laquelle:

(a) la position d'acide aminé 54 est substituée par Ser, Glu ou Thr; ou
(b) la position d'acide aminé 78 est substituée par Val; ou
(c) la position d'acide aminé 81 est substituée par Ser; ou
(d) la position d'acide aminé 191 est substituée par Asn ou His; ou
(e) la position d'acide aminé 196 est substituée par Leu ou Ala; ou
(f) la position d'acide aminé 200 est substituée par Leu; ou
(g) la position d'acide aminé 204 est substituée par Leu; ou
(h) la position d'acide aminé 263 est substituée par Pro.

**4.** Variante de cutinase selon la revendication 1, où ladite variante de cutinase a une activité de polyestérase.

**5.** Variante de cutinase selon la revendication 4, où ladite variante de cutinase a une stabilité accrue en comparaison avec la cutinase de type sauvage et comprend

(a) la substitution de Gly 73 par un acide aminé sélectionné dans le groupe consistant en Phe, Lys, Leu et Val; ou
(b) la substitution de Thr 191 par un acide aminé sélectionné dans le groupe consistant en His, Leu et Tyr; ou
(c) la substitution de Thr 78 par un acide aminé sélectionné dans le groupe consistant en Ala, Leu et Val; ou
(d) la substitution de Tyr 164 par Phe; ou
(e) la substitution de Tyr 196 par un acide aminé sélectionné dans le groupe consistant en Ala, Leu et Pro; ou
(f) la substitution de Phe 194 et la substitution de Ser 219; ou
(g) la substitution de Ala 80 par Glu.

**6.** Variante de cutinase selon la revendication 5(f), où ledit Phe 194 est substitué par un acide aminé sélectionné dans le groupe consistant en Ala, His, Lys, Leu, Asn, Pro et Gly; et où ledit Ser 219 est substitué par Gly.

**7.** Variante de cutinase selon la revendication 6, où ladite variante de cutinase a une activité de polyestérase accrue en comparaison avec une cutinase de type sauvage.

**8.** Variante de cutinase selon la revendication 4, où ladite variante de cutinase a une activité de polyestérase accrue en comparaison avec la cutinase de type sauvage et comprend

(a) la substitution de Phe 194 par Ile; ou
(b) la substitution de Phe 194 par un acide aminé sélectionné dans le groupe consistant en Lys et Leu, et la substitution de Ser 219 par Gly; ou
(c) la substitution de Phe 194 par Asn; ou
(d) la substitution de Phe 194 par un acide aminé sélectionné dans le groupe consistant en Asn, Pro et Ser, et la substitution de Ser 219 par Gly; ou
(e) la substitution de Gly 73 par un acide aminé sélectionné dans le groupe consistant en Phe et Leu; ou
(f) la substitution de Gly 75 par un acide aminé sélectionné dans le groupe consistant en Asp et Glu; ou
(g) les substitutions suivantes Ile 192 par Met; Phe 194 par Val; et Ser 219 par Gly; ou
(h) la substitution de Ser 219 par Asn, et la substitution de Phe 221 par Leu; ou
(i) la substitution de Ser 99 par Lys; ou
(j) la substitution de Thr 191 par un acide aminé sélectionné dans le groupe consistant en His, Leu et Tyr; ou
(k) la substitution de Thr 78 par Leu; ou
(l) la substitution de Tyr 196 par un acide aminé sélectionné dans le groupe consistant en Ala et Pro; ou
(m) la substitution de Arg 204 par Leu; ou
(n) la substitution de Leu 263 par Pro.

**9.** Variante de cutinase selon la revendication 4, où ladite variante de cutinase possède une stabilité accrue en comparaison avec une cutinase de type sauvage et comprend

(a) la substitution de Phe 194 par un acide aminé sélectionné dans le groupe consistant en Ile, Leu, Asn et Pro; ou
(b) la substitution de Arg 204 par Leu; ou
(c) la substitution de Leu 263 par Pro.

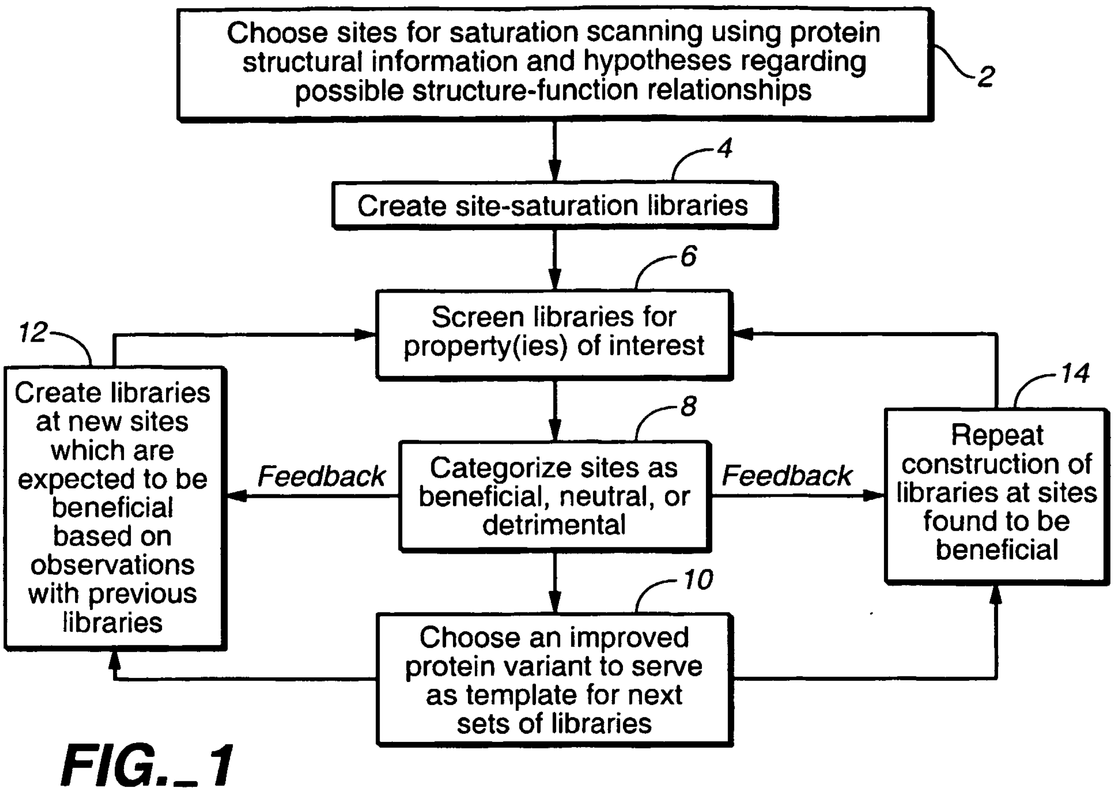
Choose sites for saturation scanning using protein structural information and hypotheses regarding possible structure-function relationships
2
4
Create site-saturation libraries
6
Screen libraries for property(ies) of interest
12
Create libraries at new sites which are expected to be beneficial based on observations with previous libraries
8
Categorize sites as beneficial, neutral, or detrimental
Feedback
Feedback
14
Repeat construction of libraries at sites found to be beneficial
10
Choose an improved protein variant to serve as template for next sets of libraries

FIG._1

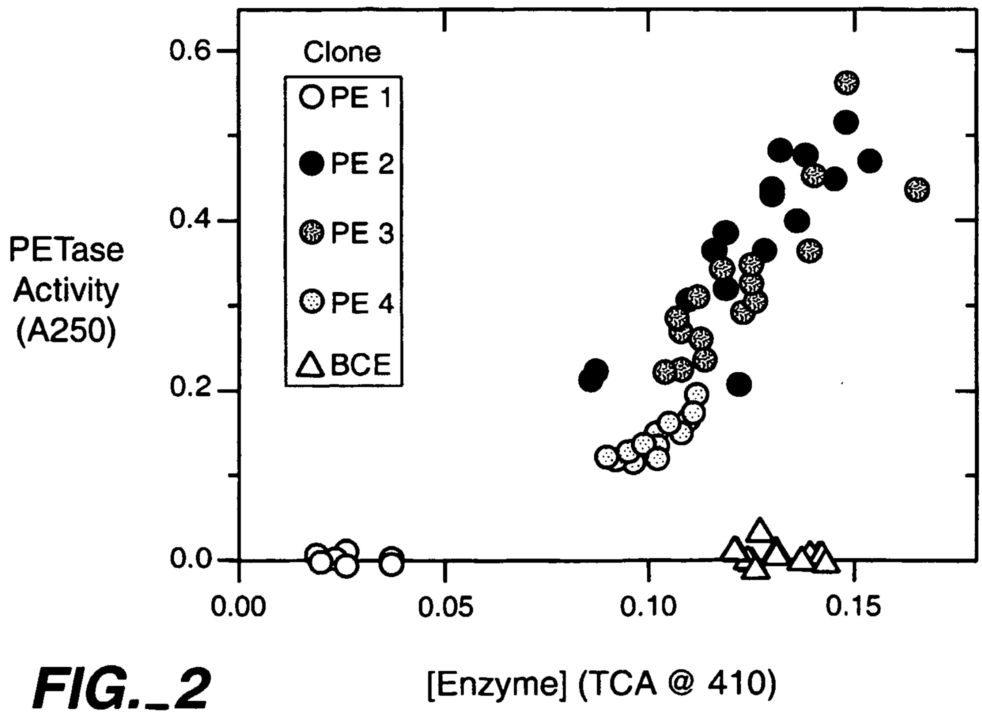
PETase Activity (A250)
0.6
0.4
0.2
0.0
Clone
PE 1
PE 2
PE 3
PE 4
BCE
0.00
0.05
0.10
0.15
[Enzyme] (TCA @ 410)

FIG._2

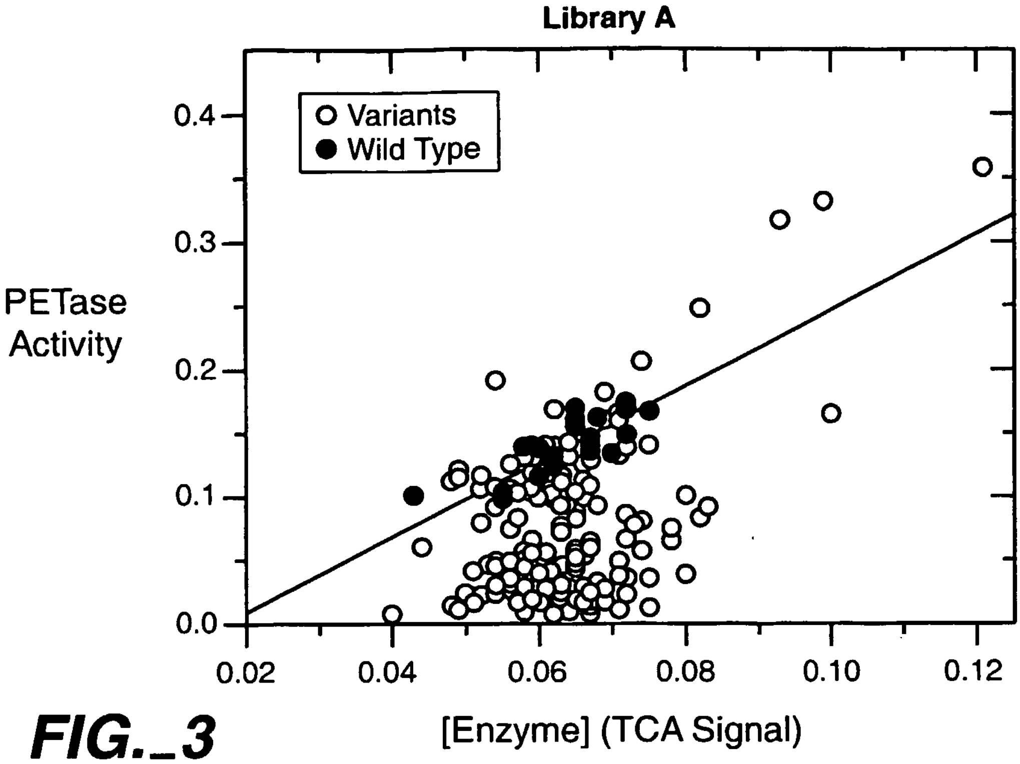
Library A
Variants
Wild Type
PETase Activity
0.4
0.3
0.2
0.1
0.0
0.02
0.04
0.06
0.08
0.10
0.12
[Enzyme] (TCA Signal)

FIG._3

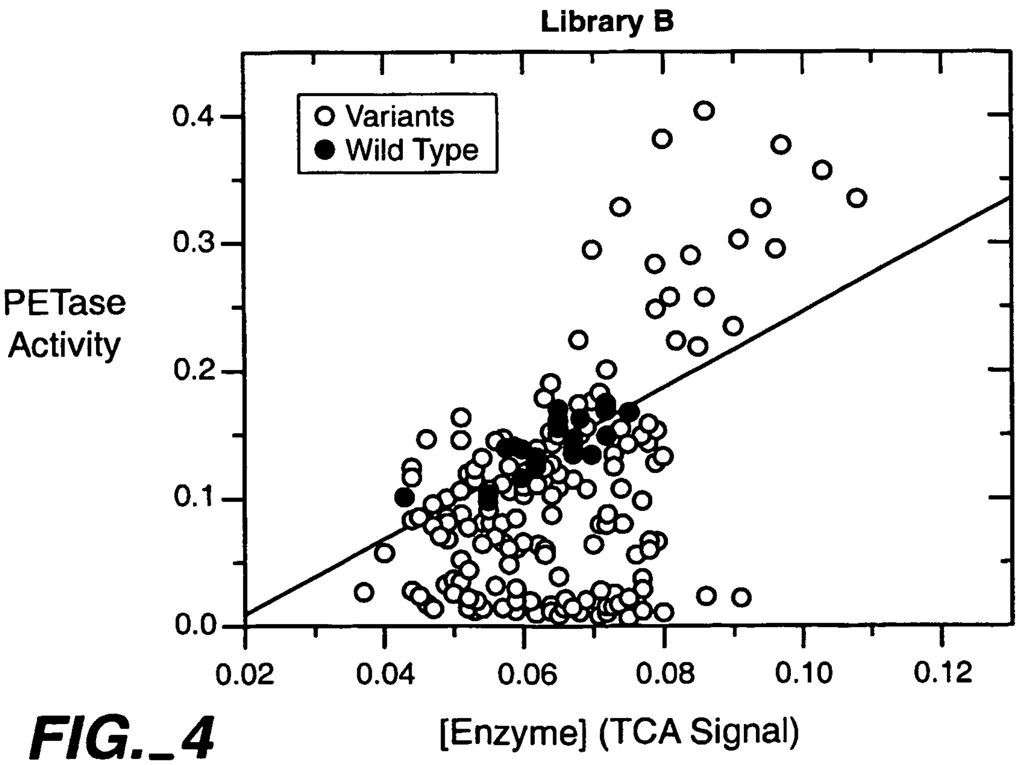
Library B
Variants
Wild Type
PETase Activity
0.4
0.3
0.2
0.1
0.0
0.02
0.04
0.06
0.08
0.10
0.12
[Enzyme] (TCA Signal)

FIG._4

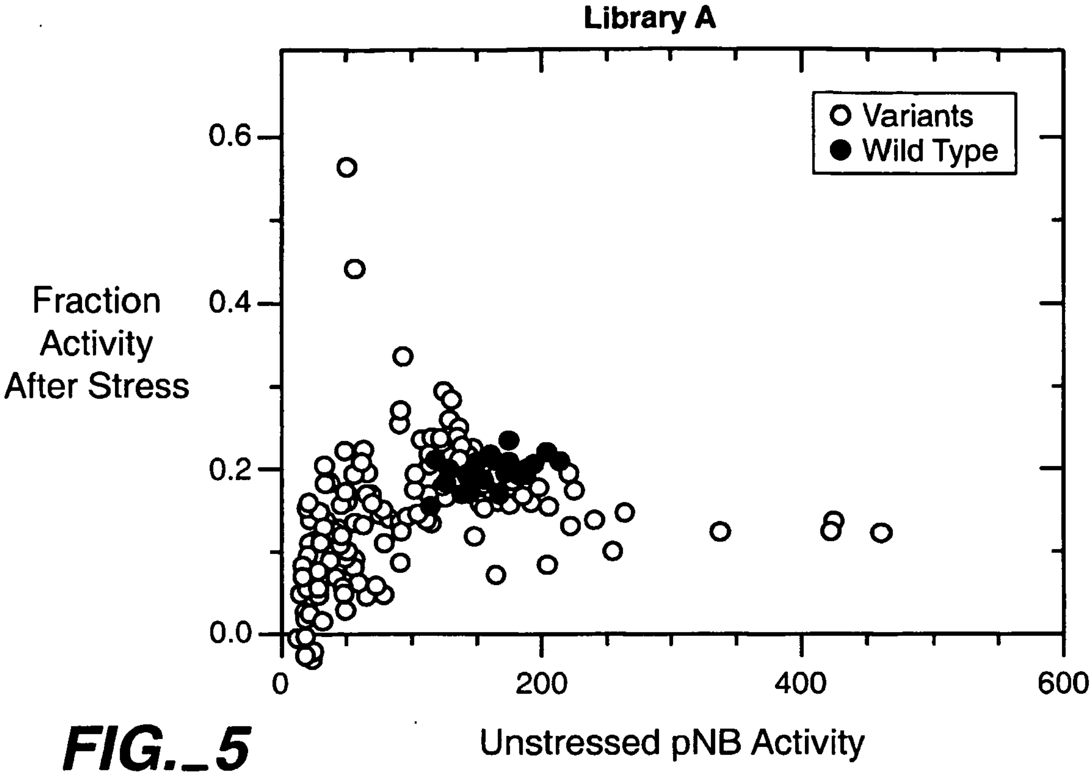
Library A
Variants
Wild Type
Fraction Activity After Stress
0.6
0.4
0.2
0.0
0
200
400
600
Unstressed pNB Activity

FIG._5

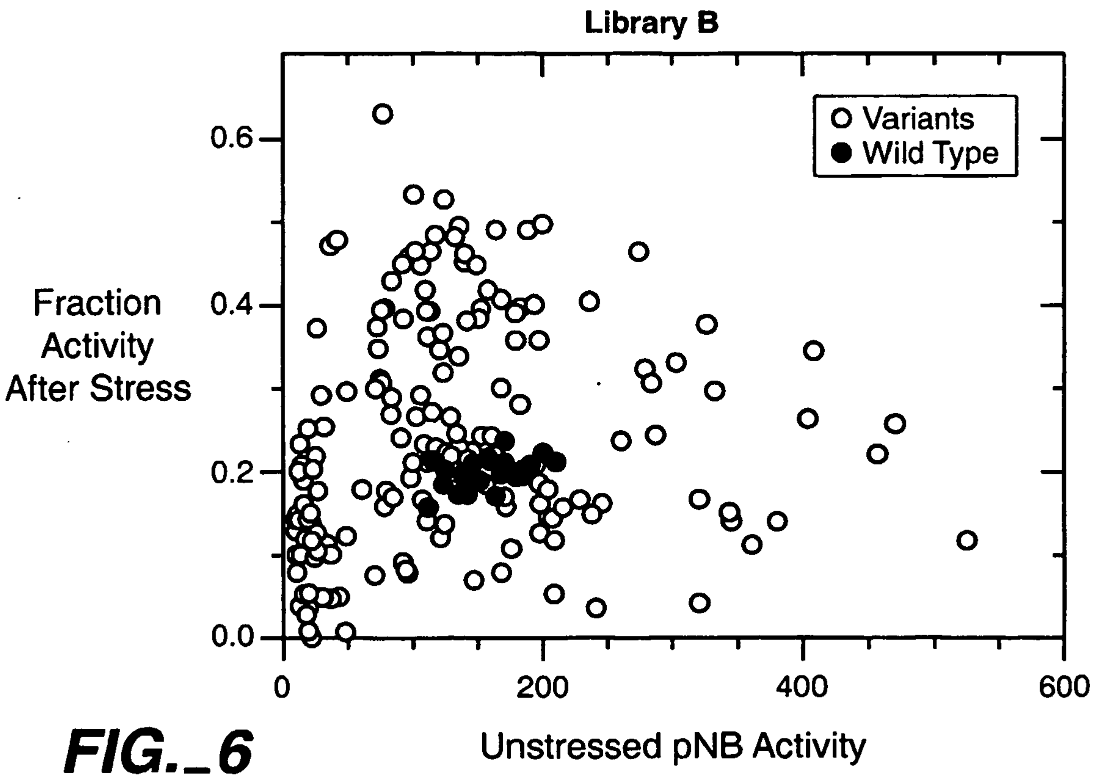
Library B
Variants
Wild Type
Fraction Activity After Stress
0.6
0.4
0.2
0.0
0
200
400
600
Unstressed pNB Activity

FIG._6

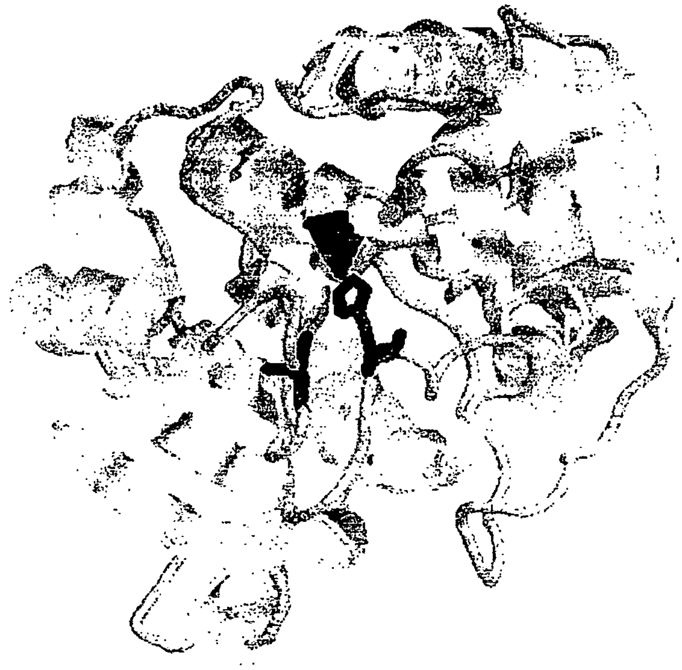

FIG._7

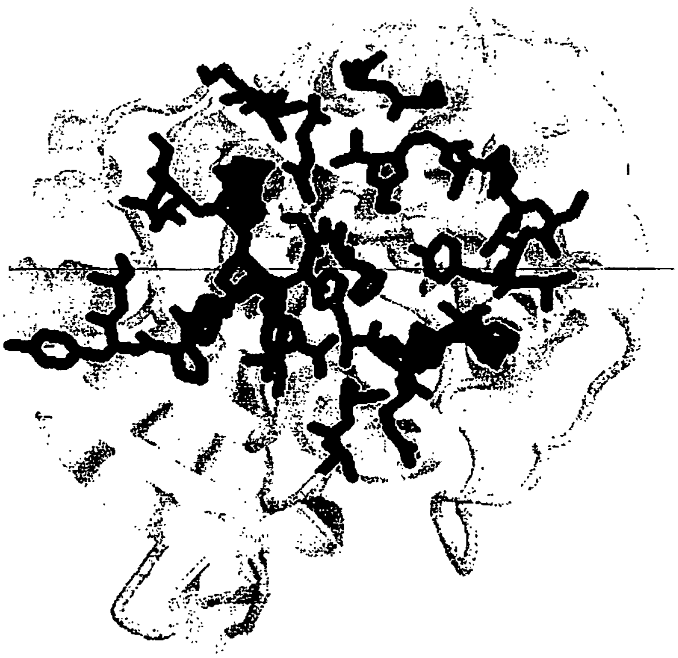

FIG._8

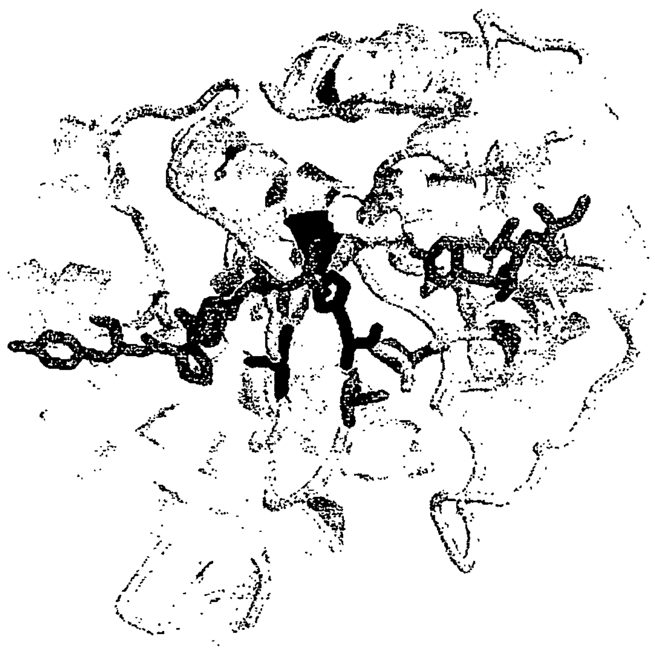

FIG._9

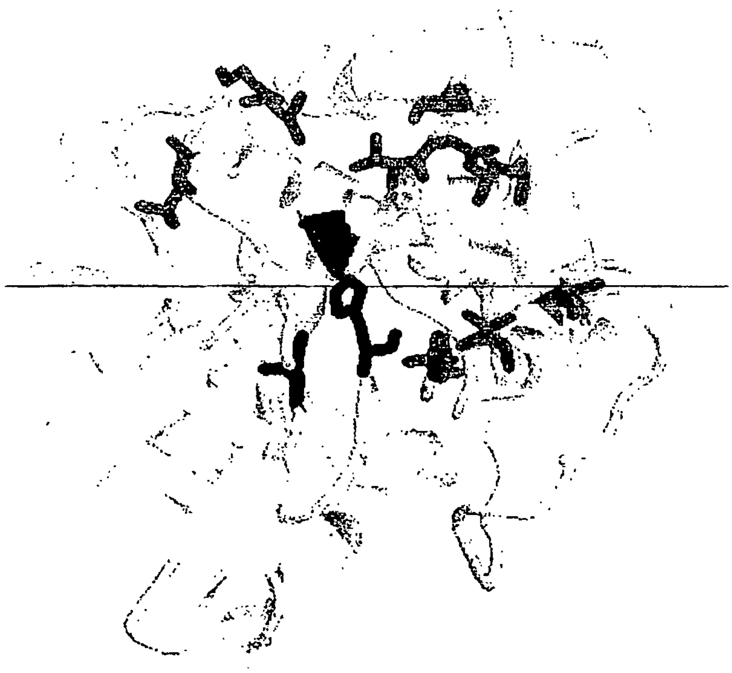

FIG._10

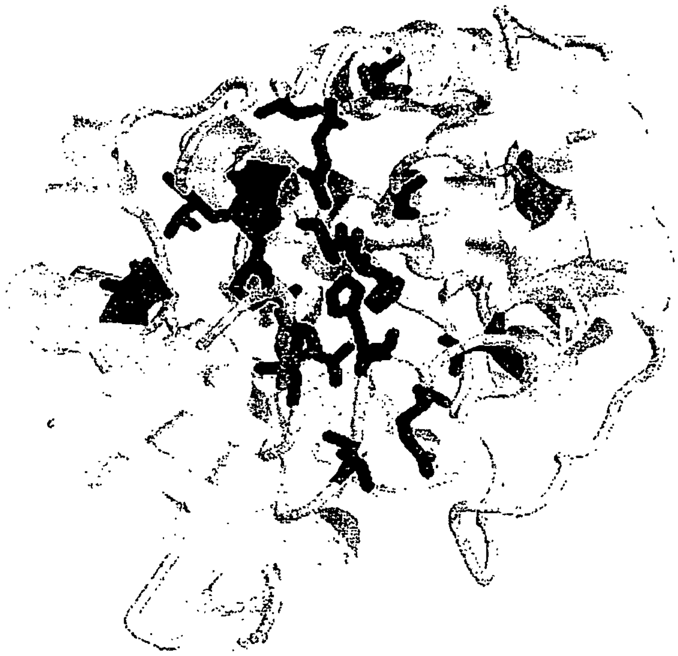

FIG._11

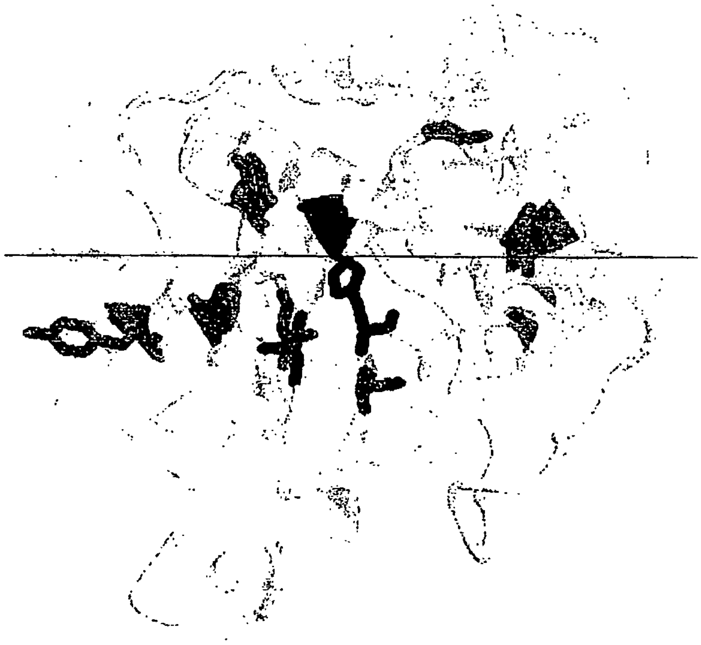

FIG._12

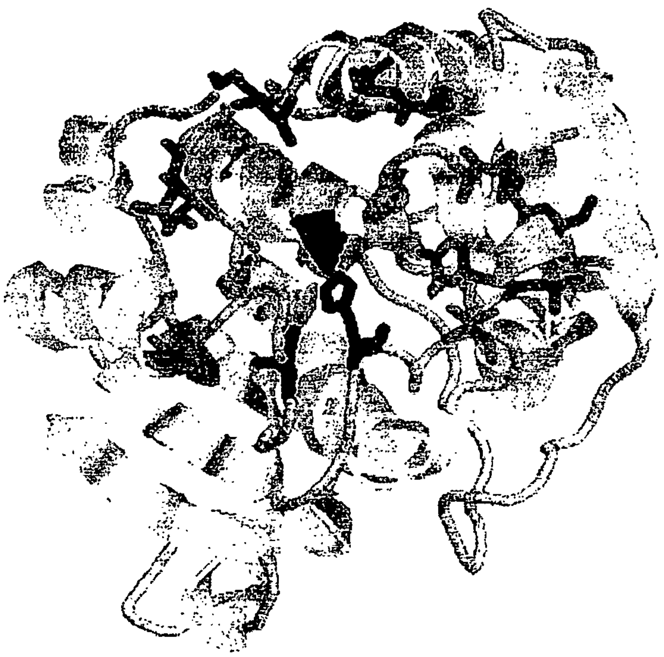

FIG._13

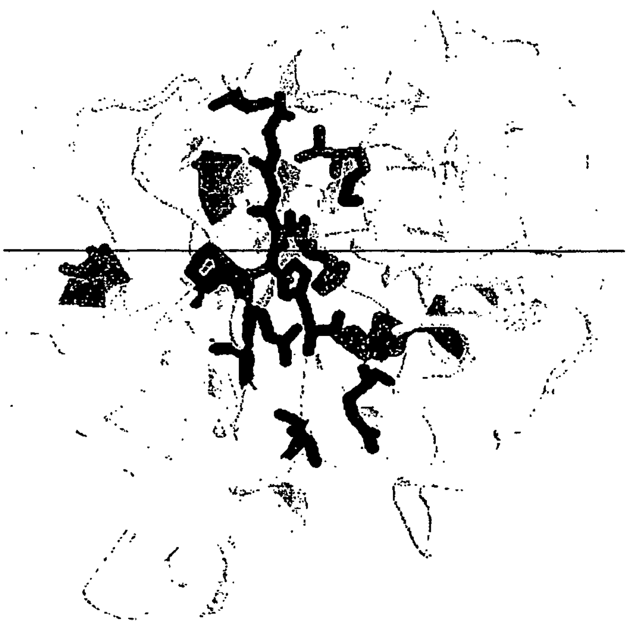

FIG._14

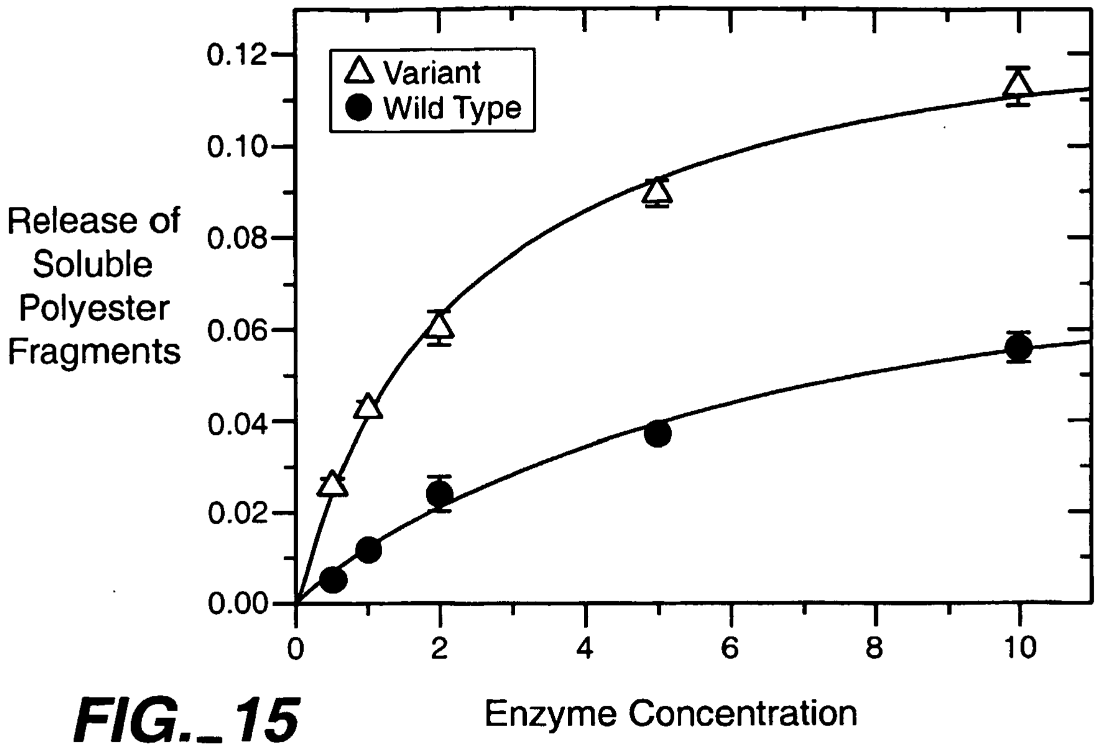
Variant
Wild Type
Release of Soluble Polyester Fragments
0.12
0.10
0.08
0.06
0.04
0.02
0.00
0
2
4
6
8
10
Enzyme Concentration

FIG._15

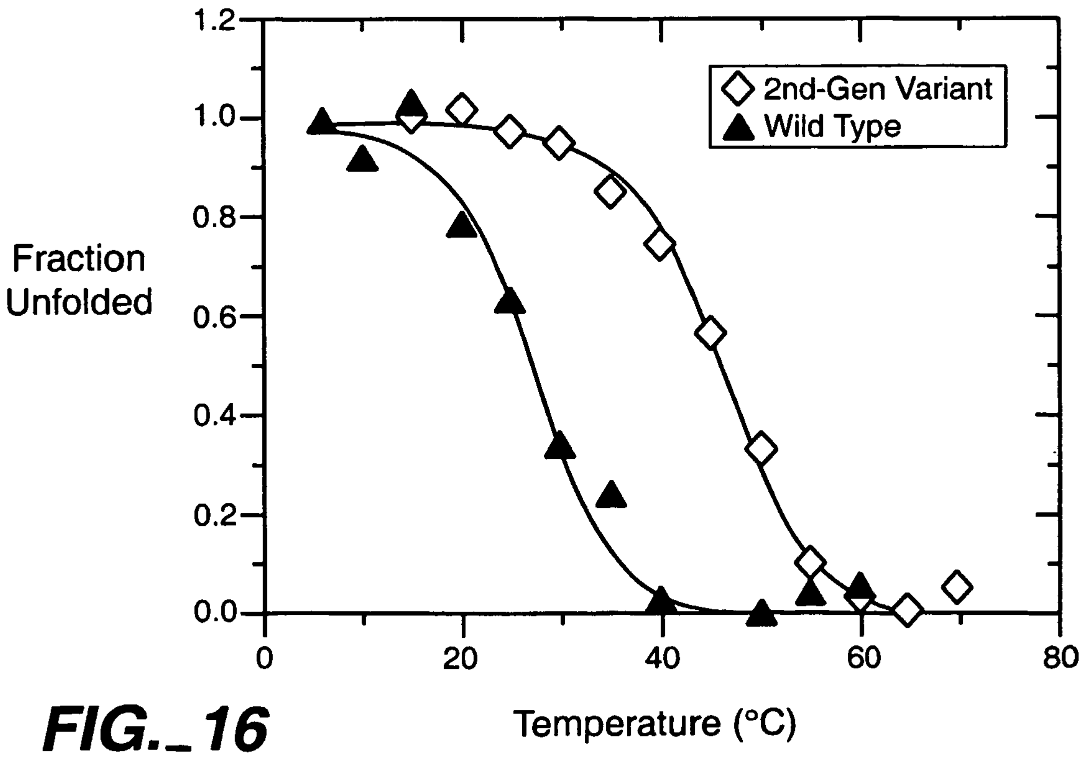
2nd-Gen Variant
Wild Type
Fraction Unfolded
1.2
1.0
0.8
0.6
0.4
0.2
0.0
0
20
40
60
80
Temperature (°C)

FIG._16

```
TGGCGGCCTCTTGCCTGTCCGTCTGTGCCACTGTCGCGGC 40
GGCTCCCCTGCCGGATACACCGGGAGCGCCATTTCCGGCT 80
GTCGCCAATTTCGACCGCAGTGGCCCCTACACCACCAGCA 120
GCCAGAGCGAGGGGCCGAGCTGTCGCATCTATCGGCCCCG 160
CGACCTGGGTCAGGGGGGCGTGCGTCATCCGGTGATTCTC 200
TGGGGCAATGGCACCGGTGCCGGGCCGTCCACCTATGCCG 240
GCTTGCTATCGCACTGGGCAAGCCACGGTTTCGTGGTGGC 280
GGCGGCGGAAACCTCCAATGCCGGTACCGGGCGGGAAATG 320
CTCGCCTGCCTGGACTATCTGGTACGTGAGAACGACACCC 360
CCTACGGCACCTATTCCGGCAAGCTCAATACCGGGCGAGT 400
CGGCACTTCTGGGCATTCCCAGGGTGGTGGCGGCTCGATC 440
ATGGCCGGGCAGGATACGAGGGTGCGTACCACGGCGCCGA 480
TCCAGCCCTACACCCTCGGCCTGGGGCACGACAGCGCCTC 520
GCAGCGGCGGCAGCAGGGGCCGATGTTCCTGATGTCCGGT 560
GGCGGTGACACCATCGCCTTTCCCTACCTCAACGCTCAGC 600
CGGTCTACCGGCGTGCCAATGTGCCGGTGTTCTGGGGCGA 640
ACGGCGTTACGTCAGCCACTTCGAGCCGGTCGGTAGCGGT 680
GGGGCCTATCGCGGCCCGAGCACGGCATGGTTCCGCTTCC 720
AGCTGATGGATGACCAAGACGCCCGCGCTACCTTCTACGG 760
CGCGCAGTGCAGTCTGTGCACCAGCCTGCTGTGGTCGGTC 800
GAGCGCCGCGGGCTTTAA                       818
```

FIG._17

FIG._18

```
TGGCGGCCTCTTGCCTGTCCGTCTGTGCCACTGTCGCGGCGGCTCCCCTGCCGGATACACCGG  63
MetAlaAlaSerCysLeuSerValCysAlaThrValAlaAlaAlaProLeuProAspThrPro

GAGCGCCATTTCCGGCTGTCGCCAATTTCGACCGCAGTGGCCCCTACACCACCAGCAGCCAGA  126
GlyAlaProPheProAlaValAlaAsnPheAspArgSerGlyProTyrThrThrSerSerGln

GCGAGGGGCCGAGCTGTCGCATCTATCGGCCCCGCGACCTGGGTCAGGGGGGCGTGCGTCATC  189
SerGluGlyProSerCysArgIleTyrArgProArgAspLeuGlyGlnGlyGlyValArgHis

CGGTGATTCTCTGGGGCAATGGCACCGGTGCCGGGCCGTCCACCTATGCCGGCTTGCTATCGC  252
ProValIleLeuTrpGlyAsnGlyThrGlyAlaGlyProSerThrTyrAlaGlyLeuLeuSer

ACTGGGCAAGCCACGGTTTCGTGGTGGCGGCGGCGGAAACCTCCAATGCCGGTACCGGGCGGG  315
HisTrpAlaSerHisGlyPheValValAlaAlaAlaGluThrSerAsnAlaGlyThrGlyArg

AAATGCTCGCCTGCCTGGACTATCTGGTACGTGAGAACGACACCCCCTACGGCACCTATTCCG  378
GluMetLeuAlaCysLeuAspTyrLeuValArgGluAsnAspThrProTyrGlyThrTyrSer

GCAAGCTCAATACCGGGCGAGTCGGCACTTCTGGGCATTCCCAGGGTGGTGGCGGCTCGATCA  441
GlyLysLeuAsnThrGlyArgValGlyThrSerGlyHisSerGlnGlyGlyGlyGlySerIle

TGGCCGGGCAGGATACGAGGGTGCGTACCACGGCGCCGATCCAGCCCTACACCCTCGGCCTGG  504
MetAlaGlyGlnAspThrArgValArgThrThrAlaProIleGlnProTyrThrLeuGlyLeu

GGCACGACAGCGCCTCGCAGCGGCGGCAGCAGGGGCCGATGTTCCTGATGTCCGGTGGCGGTG  567
GlyHisAspSerAlaSerGlnArgArgGlnGlnGlyProMetPheLeuMetSerGlyGlyGly

ACACCATCGCCTTTCCCTACCTCAACGCTCAGCCGGTCTACCGGCGTGCCAATGTGCCGGTGT  630
AspThrIleAlaPheProTyrLeuAsnAlaGlnProValTyrArgArgAlaAsnValProVal

TCTGGGGCGAACGGCGTTACGTCAGCCACTTCGAGCCGGTCGGTAGCGGTGGGGCCTATCGCG  693
PheTrpGlyGluArgArgTyrValSerHisPheGluProValGlySerGlyGlyAlaTyrArg

GCCCGAGCACGGCATGGTTCCGCTTCCAGCTGATGGATGACCAAGACGCCCGCGCTACCTTCT  756
GlyProSerThrAlaTrpPheArgPheGlnLeuMetAspAspGlnAspAlaArgAlaThrPhe

ACGGCGCGCAGTGCAGTCTGTGCACCAGCCTGCTGTGGTCGGTCGAGCGCCGCGGGCTTTAA  818
TyrGlyAlaGlnCysSerLeuCysThrSerLeuLeuTrpSerValGluArgArgGlyLeu*
```

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 0268452 A
- US 4760025 A
- US RE34606 E
- US 5204015 A
- US 5185258 A
- US 5827719 A
- WO 9414963 A
- WO 9414964 A
- WO 0005389 A
- EP 03744218 A
- US 0306908 W
- US 10091912 B
- US 10092227 B
- US 60362372 B
- US 60403921 B


### Non-patent literature cited in the description


- **Airaksinen ; Hovi.** *Nucleic Acids.Res.,* 1998, vol. 26, 576-581
- **Singleton ; Sainsbury.** Dictionary of Microbiology and Molecular Biology. John Wiley and Sons, 1994
- **Marham.** The Harper Collins Dictionary of Biology. Harper Perennial, 1991
- **Smith ; Waterman.** *Adv. Appl. Math.,* 1981, vol. 2, 482
- **Needleman ; Wunsch.** *J. Mol. Biol.,* 1970, vol. 48, 443
- **Pearson ; Lipman.** *Proc. Natl. Acad. Sci. USA,* 1988, vol. 85, 2444
- **Devereux et al.** *Nucl. Acid Res.,* 1984, vol. 12, 387-395
- **Feng ; Doolittle.** *J. Mol. Evol.,* 1987, vol. 35, 351-360
- **Higgins ; Sharp.** *CABIOS,* 1989, vol. 5, 151-153
- **Altschul et al.** *J. Mol. Biol.,* 1990, vol. 215, 403-410
- **Karlin et al.** *Proc. Natl. Acad. Sci. USA,* 1993, vol. 90, 5873-5787
- **Altschul et al.** *Meth. Enzymol.,* 1996, vol. 266, 460-480
- **Henikoff ; Henikoff.** *Proc. Natl. Acad. Sci. USA,* 1989, vol. 89, 10915
- **Henikoff et al.** *Proc. Natl. Acad Sci. USA,* 1989, vol. 89, 10915
- **Karin et al.** *Proc. Natl Acad. Sci USA,* 1993, vol. 90, 5873
- **Higgins et al.** *Gene,* 1988, vol. 73, 237-244
- **Pearson et al.** *Proc. Natl. Acad. Sci. USA,* 1988, vol. 85, 2444-2448
- Lipases. Elsevier, 1984, 471-504
- **Longhi et al.** *J. Mol. Biol.,* 1997, vol. 268, 779-799
- *Appl. Environ. Microbiol.,* 1998, vol. 64, 2794-2799
- *Proteins: Structure, Function and Genetics,* 1996, vol. 26, 442-458
- *J. Comput. Chem.,* 1996, vol. 17, 1783-1803
- *Protein Engineer.,* 1993, vol. 6, 157-165
- **Airaksinen ; Hovi.** *Nucl. Acids Res.,* 1998, vol. 26, 576-581

SEQUENCE LISTING

<110> Genencor International, Inc.

<120> High Throughput Mutagenesis Screening Method

<130> SJK/FP6240899

<140> EP 03744218.3
<141> 2003-03-05

<150> PCT/US03/06908
<151> 2003-03-05

<150> US 10/091,912
<151> 2002-03-05

<150> US 10/092,227
<151> 2002-03-05

<150> US 60/362,372
<151> 2002-03-05

<150> US 60/403,921
<151> 2002-08-15

<160> 2

<170> FastSEQ for Windows Version 4.0

<210> 1
<211> 818
<212> DNA
<213> Pseudomonas mendocina

<400> 1

```
tggcggcctc ttgcctgtcc gtctgtgcca ctgtcgcggc ggctcccctg ccggatacac      60
cgggagcgcc atttccggct gtcgccaatt tcgaccgcag tggcccctac accaccagca     120
gccagagcga ggggccgagc tgtcgcatct atcggccccg cgacctgggt caggggggcg     180
tgcgtcatcc ggtgattctc tggggcaatg gcaccggtgc cgggccgtcc acctatgccg     240
gcttgctatc gcactgggca agccacggtt tcgtggtggc ggcggcggaa acctccaatg     300
ccggtaccgg gcgggaaatg ctcgcctgcc tggactatct ggtacgtgag aacgacaccc     360
cctacggcac ctattccggc aagctcaata ccgggcgagt cggcacttct gggcattccc     420
agggtggtgg cggctcgatc atggccgggc aggatacgag ggtgcgtacc acggcgccga     480
tccagcccta caccctcggc ctggggcacg acagcgcctc gcagcggcgg cagcaggggc     540
cgatgttcct gatgtccggt ggcggtgaca ccatcgcctt tccctacctc aacgctcagc     600
cggtctaccg gcgtgccaat gtgccggtgt tctggggcga acggcgttac gtcagccact     660
tcgagccggt cggtagcggt ggggcctatc gcggcccgag cacggcatgg ttccgcttcc     720
agctgatgga tgaccaagac gcccgcgcta ccttctacgg cgcgcagtgc agtctgtgca     780
ccagcctgct gtggtcggtc gagcgccgcg ggctttaa                             818
```

<210> 2
<211> 273
<212> PRT
<213> Pseudomonas mendocina

<400> 2

```
Met Ala Ala Ser Cys Leu Ser Val Cys Ala Thr Val Ala Ala Ala Pro
 1               5                  10                  15
```

```
Leu Pro Asp Thr Pro Gly Ala Pro Phe Pro Ala Val Ala Asn Phe Asp
            20                  25                  30
Arg Ser Gly Pro Tyr Thr Thr Ser Ser Gln Ser Glu Gly Pro Ser Cys
        35                  40                  45
Arg Ile Tyr Arg Pro Arg Asp Leu Gly Gln Gly Gly Val Arg His Pro
    50                  55                  60
Val Ile Leu Trp Gly Asn Gly Thr Gly Ala Gly Pro Ser Thr Tyr Ala
65                  70                  75                  80
Gly Leu Leu Ser His Trp Ala Ser His Gly Phe Val Val Ala Ala Ala
                85                  90                  95
Glu Thr Ser Asn Ala Gly Thr Gly Arg Glu Met Leu Ala Cys Leu Asp
            100                 105                 110
Tyr Leu Val Arg Glu Asn Asp Thr Pro Tyr Gly Thr Tyr Ser Gly Lys
        115                 120                 125
Leu Asn Thr Gly Arg Val Gly Thr Ser Gly His Ser Gln Gly Gly Gly
    130                 135                 140
Gly Ser Ile Met Ala Gly Gln Asp Thr Arg Val Arg Thr Thr Ala Pro
145                 150                 155                 160
Ile Gln Pro Tyr Thr Leu Gly Leu Gly His Asp Ser Ala Ser Gln Arg
                165                 170                 175
Arg Gln Gln Gly Pro Met Phe Leu Met Ser Gly Gly Gly Asp Thr Ile
            180                 185                 190
Ala Phe Pro Tyr Leu Asn Ala Gln Pro Val Tyr Arg Arg Ala Asn Val
        195                 200                 205
Pro Val Phe Trp Gly Glu Arg Arg Tyr Val Ser His Phe Glu Pro Val
    210                 215                 220
Gly Ser Gly Gly Ala Tyr Arg Gly Pro Ser Thr Ala Trp Phe Arg Phe
225                 230                 235                 240
Gln Leu Met Asp Asp Gln Asp Ala Arg Ala Thr Phe Tyr Gly Ala Gln
                245                 250                 255
Cys Ser Leu Cys Thr Ser Leu Leu Leu Trp Ser Val Glu Arg Arg Gly
            260                 265                 270
Leu
```